# EUROPEAN PATENT APPLICATION

(11) **EP 4 501 957 A1**
(43) Date of publication of application: **05.02.2025**
(21) Application number: 23778484.8
(22) Date of filing: 31.03.2023
(51) Int. Cl.: C07K 16/18, C12N 15/13, C07K 16/46, C07K 19/00, C12N 5/10, C12N 15/63, A61K 47/68, A61K 39/395

(54) **ANTI-SERUM ALBUMIN NANOBODY AND DERIVATIVE THEREOF**

(30) Priority: 31.03.2022 CN 202210337315
(71) Applicant: Biotheus Inc., Tangjiawan Town, Xiangzhou District Zhuhai, Guangdong 519080 (CN)
(72) Inventor: WU, Fan, Zhuhai, Guangdong 519080 (CN); MIAO, Xiaoniu, Zhuhai, Guangdong 519080 (CN); LUO, Yi, Zhuhai, Guangdong 519080 (CN); CHEN, Liandi, Zhuhai, Guangdong 519080 (CN); XU, Yingda, Zhuhai, Guangdong 519080 (CN)
(74) Representative: Cabinet Nony
(86) International application number: PCT/CN2023/085559
(87) International publication number: WO 2023/186120

(57) **Abstract**

The present invention relates to a nanobody or antigen-binding fragment thereof that specifically binds to serum albumin, a derivative comprising the nanobody or the antigen-binding fragment thereof, a nucleic acid encoding the antibody or antigen-binding fragment thereof, a host cell comprising same, and related uses. Further, the present invention relates to therapeutic use of the antibody or the antigen-binding fragment thereof or the derivative.

## Description

### Technical Field

The present application relates to a nanobody or antigen-binding fragment thereof capable of specifically binding to serum albumin, a derivative containing the nanobody or antigen-binding fragment thereof, a nucleic acid encoding the nanobody or antigen-binding fragment thereof, a host cell containing the nucleic acid, and related uses thereof. In addition, the present application relates to a therapeutic use of the nanobody or antigen-binding fragment thereof, or the derivative.

### Background Art

With the development of technology, antibody drugs have long become an important disease therapy independent of chemical drugs, cell therapy, etc. In the development of antibody drugs, pharmacokinetics (PK) is an important field that describes the body's effects on antibodies, including absorption, distribution and clearance, among which half-life is an important parameter of PK. Generally, a longer half-life in vivo can improve the efficacy of antibodies, but it may also increase toxicity, and tight binding to certain targets can cause antibodies to be rapidly internalized and degraded in cells, shortening the half-life in vivo.

Prolonging the half-life of macromolecular drug in vivo can reduce the frequency of administration on the basis of increasing the duration of action of the drug itself, thereby reducing the medical burden on patients. At present, the designs to improve the long-acting effect of drugs include polyethylene glycol (PEG) modification, transferrin fusion, etc., but these chemical methods have some disadvantages. For example, PEG may shield or block the antigen-binding site of antibody, reduce the efficacy of antibody or therapeutic protein, and may even cause the loss of antibody activity.

Human serum albumin (HSA) is the most abundant protein in human plasma, accounting for about half of the serum proteins. It is soluble in water and exists primarily in monomeric form. The half-life of serum albumin in the body is about 21 days. It is composed of 585 amino acids and has a molecular weight of about 66.5KD. Human serum albumin is stable, safe and non-toxic in the body, has low immunogenicity, and thus is an ideal drug carrier. For antibody drugs, human serum albumin-enabled modification can increase the half-life of drug to improve patient compliance.

The recovery mechanism of HSA is similar to that of IgG, and is mediated by FcRn. HSA and IgG are internalized into vascular endothelial cells via pinocytosis. Within endosome, the binding of membrane-bound FcRn to HSA is facilitated at an endosomal pH of about 6.0, the binding complex then undergoes recycling to the top or bottom of endothelial cells, and the membrane-bound FcRn and HSA are rapidly dissociated and recycled under the condition of blood pH of 7.4 via exocytosis.

### Contents of the present application

The inventors of the present application have screened and obtained a series of anti-serum albumin nanobodies after extensive research. The nanobodies have high binding activity to serum albumin and cross-reactivity with human, monkey and/or mouse serum albumin. In particular, the binding of the nanobodies of the present application to serum albumin is non-pH dependent, and it can effectively participate in the FcRn-mediated serum albumin circulation, thereby prolonging the in vivo half-life of the nanobodies. Therefore, the nanobodies of the present application can be coupled (e.g., fused) to drug molecules (e.g., polypeptide drugs) to prolong the in vivo half-life of the drug molecules.

In addition, the nanobodies also have the characteristics of small molecular weight and good stability. Compared with traditional common normal antibodies, the nanobodies have many advantages in drug development, such as good tissue infiltration, flexible administration mode, high degree of humanization, and facile transformation into recombinant protein.

Based on this, the present application also provides a composition containing the nanobody or antigen-binding fragment thereof, a nucleic acid encoding the nanobody or antigen-binding fragment thereof, and a host cell containing the nucleic acid, as well as related uses thereof.

Nanobody and antigen-binding fragment thereof
Therefore, in one aspect, the present application provides a nanobody or antigen-binding fragment thereof capable of specifically binding to serum albumin, wherein the nanobody or antigen-binding fragment thereof comprises:
(a) a CDR1 having a structure selected from the group consisting of: X₁X₂X₃LX₄YYX₅ (Formula I, SEQ ID NO: 72), SGFTLDYYA (SEQ ID NO: 30), GSIWGIYH (SEQ ID NO: 37), GFTFSIYS (SEQ ID NO: 31), GSIFTFYR (SEQ ID NO: 33);
(b) a CDR2 having a structure selected from the group consisting of: IX₆SSGGX₇X₈ (Formula II, SEQ ID NO: 73), ITVDGST (SEQ ID NO: 46), ISSGGSP (SEQ ID NO: 40), ITTDTST (SEQ ID NO: 42);
(c) a CDR3 having a structure selected from the group consisting of: AAAX₉LECRTX₁₀X₁₁X₁₂X₁₃YX₁₄Y (Formula III, SEQ ID NO: 74), AAATX₁₅ECRGRSSSYDY (Formula IV, SEQ ID NO: 75), AAALLECRVRSWPSDN (SEQ ID NO: 49), AAAVLECRAAEYVNS (SEQ ID NO: 54), DIRNVGGDY (SEQ ID NO: 57), SPITSIFKA (SEQ ID NO: 48), NVRNVERDY (SEQ ID NO: 50);
   wherein,
   X₁ is selected from the group consisting of (i) amino acid residues G, R and (ii) amino acid residues that are conservative substitutions relative to (i);
   X₂ is selected from the group consisting of (i) amino acid residues F, A, S, E and (ii) amino acid residues that are conservative substitutions relative to (i);
   X₃ is selected from the group consisting of (i) amino acid residues T, Q and (ii) amino acid residues that are conservative substitutions relative to (i);
   X₄ is selected from the group consisting of (i) amino acid residues D, E and (ii) amino acid residues that are conservative substitutions relative to (i);
   X₅ is selected from the group consisting of (i) amino acid residue A and (ii) amino acid residues that are conservative substitutions relative to (i);
   X₆ is selected from the group consisting of (i) amino acid residues A, S and (ii) amino acid residues that are conservative substitutions relative to (i);
   X₇ is selected from the group consisting of (i) amino acid residues A, S and (ii) amino acid residues that are conservative substitutions relative to (i);
   X₈ is selected from the group consisting of (i) amino acid residue T and (ii) amino acid residues that are conservative substitutions relative to (i);
   X₉ is selected from the group consisting of (i) amino acid residues T, V and (ii) amino acid residues that are conservative substitutions relative to (i);
   X₁₀ is selected from the group consisting of (i) amino acid residues T, V and (ii) amino acid residues that are conservative substitutions relative to (i);
   X₁₁ is selected from the group consisting of (i) amino acid residues V, I, L and (ii) amino acid residues that are conservative substitutions relative to (i);
   X₁₂ is selected from the group consisting of (i) amino acid residues R, V, T and (ii) amino acid residues that are conservative substitutions relative to (i);
   X₁₃ is selected from the group consisting of (i) amino acid residues G, E and (ii) amino acid residues that are conservative substitutions relative to (i);
   X₁₄ is selected from the group consisting of (i) amino acid residues D, A and (ii) amino acid residues that are conservative substitutions relative to (i);
   X₁₅ is selected from the group consisting of (i) amino acid residues L, F and (ii) amino acid residues that are conservative substitutions relative to (i).

In certain embodiments, the nanobody or antigen-binding fragment thereof comprises a CDR1 as set forth in any one of SEQ ID NOs: 30-38; a CDR2 as set forth in any one of SEQ ID NOs: 39-46; and a CDR3 as set forth in any one of SEQ ID NOs: 47-59.

In certain embodiments, the nanobody or antigen-binding fragment thereof comprises:
(a) a CDR1 having a structure as shown in X₁X₂X₃LX₄YYX₅ (Formula I, SEQ ID NO: 72) or SGFTLDYYA (SEQ ID NO: 30);
(b) a CDR2 having a structure as shown in IX₆SSGGX₇X₈ (Formula II, SEQ ID NO: 73);
(c) a CDR3 having a structure selected from the group consisting of: AAAX₉LECRTX₁₀X₁₁X₁₂X₁₃YX₁₄Y (Formula III, SEQ ID NO: 74), AAATX₁₅ECRGRSSSYDY (Formula IV, SEQ ID NO: 75), AAALLECRVRSWPSDN (SEQ ID NO: 49), AAAVLECRAAEYVNS (SEQ ID NO: 54);
   wherein,
   X₁ is selected from the group consisting of (i) amino acid residues G, R and (ii) amino acid residues that are conservative substitutions relative to (i);
   X₂ is selected from the group consisting of (i) amino acid residues F, A, S, E and (ii) amino acid residues that are conservative substitutions relative to (i);
   X₃ is selected from the group consisting of (i) amino acid residues T, Q and (ii) amino acid residues that are conservative substitutions relative to (i);
   X₄ is selected from the group consisting of (i) amino acid residues D, E and (ii) amino acid residues that are conservative substitutions relative to (i);
   X₅ is selected from the group consisting of (i) amino acid residue A and (ii) amino acid residues that are conservative substitutions relative to (i);
   X₆ is selected from the group consisting of (i) amino acid residues A, S and (ii) amino acid residues that are conservative substitutions relative to (i);
   X₇ is selected from the group consisting of (i) amino acid residues A, S and (ii) amino acid residues that are conservative substitutions relative to (i);
   X₈ is selected from the group consisting of (i) amino acid residue T and (ii) amino acid residues that are conservative substitutions relative to (i);
   X₉ is selected from the group consisting of (i) amino acid residues T, V and (ii) amino acid residues that are conservative substitutions relative to (i);
   X₁₀ is selected from the group consisting of (i) amino acid residues T, V and (ii) amino acid residues that are conservative substitutions relative to (i);
   X₁₁ is selected from the group consisting of (i) amino acid residues V, I, L and (ii) amino acid residues that are conservative substitutions relative to (i);
   X₁₂ is selected from the group consisting of (i) amino acid residues R, V, T and (ii) amino acid residues that are conservative substitutions relative to (i);
   X₁₃ is selected from the group consisting of (i) amino acid residues G, E and (ii) amino acid residues that are conservative substitutions relative to (i);
   X₁₄ is selected from the group consisting of (i) amino acid residues D, A and (ii) amino acid residues that are conservative substitutions relative to (i);
   X₁₅ is selected from the group consisting of (i) amino acid residues L, F and (ii) amino acid residues that are conservative substitutions relative to (i).

In certain embodiments, X₁ is selected from the group consisting of amino acid residues G and R; X₂ is selected from the group consisting of amino acid residues F, A, S and E; X₃ is selected from the group consisting of amino acid residues T and Q; X₄ is selected from the group consisting of amino acid residues D and E; X₅ is amino acid residue A; X₆ is selected from the group consisting of amino acid residues A and S; X₇ is selected from the group consisting of amino acid residues A and S; X₈ is amino acid residue T; X₉ is selected from the group consisting of amino acid residues T and V; X₁₀ is selected from the group consisting of amino acid residues T and V; X₁₁ is selected from the group consisting of amino acid residues V, I and L; X₁₂ is selected from the group consisting of amino acid residues R, V and T; X₁₃ is selected from the group consisting of amino acid residues G and E; X₁₄ is selected from the group consisting of amino acid residues D and A; X₁₅ is selected from the group consisting of amino acid residues L and F.

In certain embodiments, the nanobody or antigen-binding fragment thereof comprises: a CDR1 as set forth in any one of SEQ ID NOs: 30, 32, 34-36, 38; a CDR2 as set forth in any one of SEQ ID NOs: 39, 41, 43-45; and a CDR3 as set forth in any one of SEQ ID NOs: 47, 49, 51-56, 58-59.

In certain embodiments, the nanobody or antigen-binding fragment thereof comprises:
(a) a CDR1, which has a structure as shown in X₁X₂X₃LX₄YYX₅ (Formula I, SEQ ID NO: 72) or SGFTLDYYA (SEQ ID NO: 30);
(b) a CDR2, which has a structure as shown in IX₆SSGGX₇X₈ (Formula II, SEQ ID NO: 73);
(c) a CDR3, which has a structure selected from the group consisting of AAAX₉LECRTX₁₀X₁₁X₁₂X₁₃YX₁₄Y (Formula III, SEQ ID NO: 74), AAATX₁₅ECRGRSSSYDY (Formula IV, SEQ ID NO: 75), AAALLECRVRSWPSDN (SEQ ID NO: 49), AAAVLECRAAEYVNS (SEQ ID NO: 54);
   wherein,
   X₁ is selected from the group consisting of (i) amino acid residues G, R and (ii) amino acid residues that are conservative substitutions relative to (i);
   X₂ is selected from the group consisting of (i) amino acid residues F, E, S and (ii) amino acid residues that are conservative substitutions relative to (i);
   X₃ is selected from the group consisting of (i) amino acid residue T and (ii) amino acid residues that are conservative substitutions relative to (i);
   X₄ is selected from the group consisting of (i) amino acid residue D and (ii) amino acid residues that are conservative substitutions relative to (i);
   X₅ is selected from the group consisting of (i) amino acid residue A and (ii) amino acid residues that are conservative substitutions relative to (i);
   X₆ is selected from the group consisting of (i) amino acid residues A, S and (ii) amino acid residues that are conservative substitutions relative to (i);
   X₇ is selected from the group consisting of (i) amino acid residues A, S and (ii) amino acid residues that are conservative substitutions relative to (i);
   X₈ is selected from the group consisting of (i) amino acid residue T and (ii) amino acid residues that are conservative substitutions relative to (i);
   X₉ is selected from the group consisting of (i) amino acid residues T, V and (ii) amino acid residues that are conservative substitutions relative to (i);
   X₁₀ is selected from the group consisting of (i) amino acid residue V and (ii) amino acid residues that are conservative substitutions relative to (i);
   X₁₁ is selected from the group consisting of (i) amino acid residues V, I and (ii) amino acid residues that are conservative substitutions relative to (i);
   X₁₂ is selected from the group consisting of (i) amino acid residues R, V and (ii) amino acid residues that are conservative substitutions relative to (i);
   X₁₃ is selected from the group consisting of (i) amino acid residues G, E and (ii) amino acid residues that are conservative substitutions relative to (i);
   X₁₄ is selected from the group consisting of (i) amino acid residue D and (ii) amino acid residues that are conservative substitutions relative to (i);
   X₁₅ is selected from the group consisting of (i) amino acid residues L, F and (ii) amino acid residues that are conservative substitutions relative to (i).

In certain embodiments, X₁ is selected from the group consisting of amino acid residues G and R; X₂ is selected from the group consisting of amino acid residues F, S and E; X₃ is amino acid residue T; X₄ is amino acid residue D; X₅ is amino acid residue A; X₆ is selected from the group consisting of amino acid residues A and S; X₇ is selected from the group consisting of amino acid residues A and S; X₈ is amino acid residue T; X₉ is selected from the group consisting of amino acid residues T and V; X₁₀ is amino acid residue V; ₁₁ is selected from the group consisting of amino acid residues V and I; X₁₂ is selected from the group consisting of amino acid residues R and V; X₁₃ is selected from the group consisting of amino acid residues G and E; X₁₄ is amino acid residue D; X₁₅ is selected from the group consisting of amino acid residues L and F.

In certain embodiments, the nanobody or antigen-binding fragment thereof comprises: a CDR1 as set forth in any one of SEQ ID NOs: 30, 32, 34-36; a CDR2 as set forth in any one of SEQ ID NOs: 39, 41, 43-45; and a CDR3 as set forth in any one of SEQ ID NOs: 47, 49, 51, 52, 54-56, 58.

In certain embodiments, the nanobody or antigen-binding fragment thereof comprises:
(a) a CDR1, which has: a sequence as set forth in SEQ ID NO: 32, or a sequence having a substitution, deletion or addition of one or several amino acids (e.g., a substitution, deletion or addition of 1, 2 or 3 amino acids) as compared to the sequence as set forth in SEQ ID NO: 32;
(b) a CDR2, which has: a sequence as set forth in SEQ ID NO: 41, or a sequence having a substitution, deletion or addition of one or several amino acids (e.g., a substitution, deletion or addition of 1, 2 or 3 amino acids) as compared to the sequence as set forth in SEQ ID NO: 41; and
(c) a CDR3, which has: a sequence as set forth in SEQ ID NO: 49, or a sequence having a substitution, deletion or addition of one or several amino acids (e.g., a substitution, deletion or addition of 1, 2 or 3 amino acids) as compared to the sequence as set forth in SEQ ID NO: 49.

In certain embodiments, the substitution is a conservative substitution.

In certain embodiments, the nanobody or antigen-binding fragment thereof comprises: a CDR1 as set forth in SEQ ID NO: 32, a CDR2 as set forth in SEQ ID NO: 41, and a CDR3 as set forth in SEQ ID NO: 49.

In certain embodiments, the nanobody or antigen-binding fragment thereof comprises:
(a) a CDR1, which has: a sequence as set forth in SEQ ID NO: 32 or 34, or a sequence having a substitution, deletion or addition of one or several amino acids (e.g., a substitution, deletion or addition of 1, 2 or 3 amino acids) as compared to the sequence as set forth in SEQ ID NO: 32 or 34;
(b) a CDR2, which has: a sequence as set forth in SEQ ID NO: 43, or a sequence having a substitution, deletion or addition of one or several amino acids (e.g., a substitution, deletion or addition of 1, 2 or 3 amino acids) as compared to the sequence as set forth in SEQ ID NO: 43; and
(c) a CDR3, which has: a sequence as set forth in SEQ ID NO: 52, or a sequence having a substitution, deletion or addition of one or several amino acids (e.g., a substitution, deletion or addition of 1, 2 or 3 amino acids) as compared to the sequence as set forth in SEQ ID NO: 52.

In certain embodiments, the substitution is a conservative substitution.

In certain embodiments, the nanobody or antigen-binding fragment thereof comprises:
(a) a CDR1 having a structure as shown in X₁X₂X₃LX₄YYX₅ (Formula I, SEQ ID NO: 72), wherein X₁ is amino acid residue G; X₂ is selected from the group consisting of (i) amino acid residues F, E and (ii) amino acid residues that are conservative substitutions relative to (i); X₃ is amino acid residue T; X₄ is amino acid residue D; X₅ is amino acid residue A;
(b) a CDR2 having a structure as shown in IASSGGST (SEQ ID NO: 43);
(c) a CDR3 having a structure as shown in AAAVLECRTVVRGYDY (SEQ ID NO: 52).

In certain embodiments, the nanobody or antigen-binding fragment thereof comprises: a CDR1 as set forth in SEQ ID NO: 32 or 34; a CDR2 as set forth in SEQ ID NO: 43; and a CDR3 as set forth in SEQ ID NO: 52.

In certain embodiments, the nanobody or antigen-binding fragment thereof comprises:
(a) a CDR1, which has: a sequence as set forth in SEQ ID NO: 37, or a sequence having a substitution, deletion or addition of one or several amino acids (e.g., a substitution, deletion or addition of 1, 2 or 3 amino acids) as compared to the sequence as set forth in SEQ ID NO: 37;
(b) a CDR2, which has: a sequence as set forth in SEQ ID NO: 46, or a sequence having a substitution, deletion or addition of one or several amino acids (e.g., a substitution, deletion or addition of 1, 2 or 3 amino acids) as compared to the sequence as set forth in SEQ ID NO: 46; and
(c) a CDR3, which has: a sequence as set forth in SEQ ID NO: 57, or a sequence having a substitution, deletion or addition of one or several amino acids (e.g., a substitution, deletion or addition of 1, 2 or 3 amino acids) as compared to the sequence as set forth in SEQ ID NO: 57.

In certain embodiments, the substitution is a conservative substitution.

In certain embodiments, the nanobody or antigen-binding fragment thereof comprises: a CDR1 as set forth in SEQ ID NO: 37, a CDR2 as set forth in SEQ ID NO: 46, and a CDR3 as set forth in SEQ ID NO: 57.

In certain embodiments, the nanobody or antigen-binding fragment thereof comprises:
(a) a CDR1, which has: a sequence as set forth in SEQ ID NO: 31, or a sequence having a substitution, deletion or addition of one or several amino acids (e.g., a substitution, deletion or addition of 1, 2 or 3 amino acids) as compared to the sequence as set forth in SEQ ID NO: 31;
(b) a CDR2, which has: a sequence as set forth in SEQ ID NO: 40, or a sequence having a substitution, deletion or addition of one or several amino acids (e.g., a substitution, deletion or addition of 1, 2 or 3 amino acids) as compared to the sequence as set forth in SEQ ID NO: 40; and
(c) a CDR3, which has: a sequence as set forth in SEQ ID NO: 48, or a sequence having a substitution, deletion or addition of one or several amino acids (e.g., a substitution, deletion or addition of 1, 2 or 3 amino acids) as compared to the sequence as set forth in SEQ ID NO: 48.

In certain embodiments, the substitution is a conservative substitution.

In certain embodiments, the nanobody or antigen-binding fragment thereof comprises: a CDR1 as set forth in SEQ ID NO: 31, a CDR2 as set forth in SEQ ID NO: 40, and a CDR3 as set forth in SEQ ID NO: 48.

In certain embodiments, the nanobody or antigen-binding fragment thereof comprises:
(a) a CDR1, which has: a sequence as set forth in SEQ ID NO: 33, or a sequence having a substitution, deletion or addition of one or several amino acids (e.g., a substitution, deletion or addition of 1, 2 or 3 amino acids) as compared to the sequence as set forth in SEQ ID NO: 33;
(b) a CDR2, which has: a sequence as set forth in SEQ ID NO: 42, or a sequence having a substitution, deletion or addition of one or several amino acids (e.g., a substitution, deletion or addition of 1, 2 or 3 amino acids) as compared to the sequence as set forth in SEQ ID NO: 42; and
(c) a CDR3, which has: a sequence as set forth in SEQ ID NO: 50, or a sequence having a substitution, deletion or addition of one or several amino acids (e.g., a substitution, deletion or addition of 1, 2 or 3 amino acids) as compared to the sequence as set forth in SEQ ID NO: 50.

In certain embodiments, the substitution is a conservative substitution.

In certain embodiments, the nanobody or antigen-binding fragment thereof comprises: a CDR1 as set forth in SEQ ID NO: 33, a CDR2 as set forth in SEQ ID NO: 42, and a CDR3 as set forth in SEQ ID NO: 50.

In certain embodiments, the nanobody or antigen-binding fragment thereof comprises:
(1) a CDR1 as set forth in SEQ ID NO: 30; a CDR2 as set forth in SEQ ID NO: 39; and a CDR3 as set forth in SEQ ID NO: 47;
(2) a CDR1 as set forth in SEQ ID NO: 31; a CDR2 as set forth in SEQ ID NO: 40; and a CDR3 as set forth in SEQ ID NO: 48;
(3) a CDR1 as set forth in SEQ ID NO: 32; a CDR2 as set forth in SEQ ID NO: 41; and a CDR3 as set forth in SEQ ID NO: 49;
(4) a CDR1 as set forth in SEQ ID NO: 33; a CDR2 as set forth in SEQ ID NO: 42; and a CDR3 as set forth in SEQ ID NO: 50;
(5) a CDR1 as set forth in SEQ ID NO: 32; a CDR2 as set forth in SEQ ID NO: 43; and a CDR3 as set forth in SEQ ID NO: 51;
(6) a CDR1 as set forth in SEQ ID NO: 32 or 34; a CDR2 as set forth in SEQ ID NO: 43; and a CDR3 as set forth in SEQ ID NO: 52;
(7) a CDR1 as set forth in SEQ ID NO: 35; a CDR2 as set forth in SEQ ID NO: 43; and a CDR3 as set forth in SEQ ID NO: 52;
(8) a CDR1 as set forth in SEQ ID NO: 32; CDR2 as set forth in SEQ ID NO: 43; and CDR3 as set forth in SEQ ID NO: 53;
(9) a CDR1 as set forth in SEQ ID NO: 36; a CDR2 as set forth in SEQ ID NO: 41; and a CDR3 as set forth in SEQ ID NO: 54;
(10) a CDR1 as set forth in SEQ ID NO: 35; a CDR2 as set forth in SEQ ID NO: 44; and a CDR3 as set forth in SEQ ID NO: 55;
(11) a CDR1 as set forth in SEQ ID NO: 32; a CDR2 as set forth in SEQ ID NO: 45; and, a CDR3 as set forth in SEQ ID NO: 56;
(12) a CDR1 as set forth in SEQ ID NO: 32; a CDR2 as set forth in SEQ ID NO: 41; and, a CDR3 as set forth in SEQ ID NO: 52;
(13) a CDR1 as set forth in SEQ ID NO: 37; a CDR2 as set forth in SEQ ID NO: 46; and, a CDR3 as set forth in SEQ ID NO: 57;
(14) a CDR1 as set forth in SEQ ID NO: 32; CDR2 as set forth in SEQ ID NO: 41; and, CDR3 as set forth in SEQ ID NO: 58; or
(15) a CDR1 as set forth in SEQ ID NO: 38; a CDR2 as set forth in SEQ ID NO: 41; and, a CDR3 as set forth in SEQ ID NO: 59.

In certain embodiments, the nanobody or antigen-binding fragment thereof comprises an amino acid sequence selected from the group consisting of:
(i) a sequence as set forth in any one of SEQ ID NOs: 1-3, 7-9, 14-24, 28-29;
(ii) a sequence having a substitution, deletion or addition of one or several amino acids (e.g., a substitution, deletion or addition of 1, 2, 3, 4 or 5 amino acids) as compared to the sequence as set forth in any one of SEQ ID NOs: 1-3, 7-9, 14-24, 28-29; and
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 1-3, 7-9, 14-24, 28-29.

In certain embodiments, the substitution is a conservative substitution.

In certain embodiments, the nanobody or antigen-binding fragment thereof is humanized.

In certain embodiments, the nanobody or antigen-binding fragment thereof further comprises a heavy chain framework region of a human immunoglobulin (e.g., a heavy chain framework region contained in an amino acid sequence encoded by a human heavy chain germline antibody gene), the heavy chain framework region optionally comprising one or more (e.g., 1, 2, 3, 4, 5, 6, 7, 8, 9 or 10) back mutations from human residues to camelid residues.

In certain embodiments, the nanobody or antigen-binding fragment thereof comprises an amino acid sequence selected from the group consisting of:
(i) a sequence as set forth in any one of SEQ ID NOs: 4-6, 10-13, 25-27, 78;
(ii) a sequence having a substitution, deletion or addition of one or several amino acids (e.g., a substitution, deletion or addition of 1, 2, 3, 4 or 5 amino acids) as compared to the sequence as set forth in any one of SEQ ID NOs: 4-6, 10-13, 25-27, 78; and
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 4-6, 10-13, 25-27, 78.

In certain embodiments, the substitution is a conservative substitution.

In certain embodiments, the serum albumin is selected from the group consisting of human serum albumin (HSA), mouse serum albumin (MSA) and/or cynomolgus serum albumin (CSA).

In certain embodiments, the binding of the nanobody or antigen-binding fragment thereof to the serum albumin is pH-independent.

In certain embodiments, the nanobody or antigen-binding fragment thereof is capable of specifically binding to the serum albumin in the pH range of 5 to 8 (e.g., 5.5 to 7.4).

### Polypeptide construct

In another aspect, the present application also provides a polypeptide construct capable of specifically binding to serum albumin, which comprises the nanobody or antigen-binding fragment thereof as described above, and an immunoglobulin Fc domain.

As used herein, the Fc domain is also referred to as the Fc region, which refers to a portion of the heavy chain constant region comprising CH2 and CH3. In some embodiments, the Fc domain comprises a hinge, CH2 and CH3. When the Fc domain comprises a hinge, the hinge regulates the dimerization between two Fc-containing polypeptides. The Fc domain can be any isotype of antibody heavy chain constant region. In some embodiments, the Fc domain is of IgG1, IgG2, IgG3, or IgG4.

In certain embodiments, the Fc domain contained in the polypeptide construct of the present application is a natural Fc region, which comprises an amino acid sequence consistent with the amino acid sequence of the Fc region found in the nature. For example, the Fc domain can be a human IgG1 Fc region with a natural sequence, a human IgG2 Fc region with a natural sequence, a human IgG3 Fc region with a natural sequence, or a human IgG4 Fc region with a natural sequence. The natural Fc region may have effector functions. Exemplary "effector functions" include binding to Fc receptor; Clq binding, and complement dependent cytotoxicity (CDC); antibody-dependent cell-mediated cytotoxicity (ADCC); phagocytosis; downregulation of cell surface receptor (e.g., B cell receptor); and B cell activation, etc. Replacing at least one amino acid residue in the natural Fc region with a different residue or chemical modification to at least one amino acid residue can produce functional changes, such as changes in the affinity of antibody for effector ligands (e.g., FcR or complement C1q), thereby changing (e.g., reducing or enhancing) the effector function.

Therefore, in certain embodiments, the Fc domain contained in the polypeptide construct of the present application may also be a variant Fc region, which may comprises one or more (e.g., 1-10, such as 1-5) amino acid mutations or chemical modifications as compared to the natural Fc region so as to change one or more of the following properties of the antibody of the present application: Fc receptor binding, antibody glycosylation, number of cysteine residues, effector cell function or complement function, etc.

In certain embodiments, the Fc domain contained in the polypeptide construct of the present application has ADCC activity. In certain embodiments, the Fc domain contained in the polypeptide construct of the present application does not have ADCC activity.

In certain embodiments, the immunoglobulin Fc domain is connected to the N-terminal and/or C-terminal (e.g., C-terminal) of the nanobody or antigen-binding fragment thereof, optionally via a peptide linker.

In certain embodiments, the immunoglobulin Fc domain is a Fc domain of IgG (e.g., a Fc domain of IgG1).

In certain embodiments, the immunoglobulin Fc domain comprises a sequence as set forth in SEQ ID NO: 68, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or several amino acids (e.g., a substitution, deletion, or addition of 1, 2, 3, 4, or 5 amino acids) as compared thereto.

In certain embodiments, the serum albumin is selected from the group consisting of human serum albumin (HSA), mouse serum albumin (MSA) and/or cynomolgus serum albumin (CSA).

In certain embodiments, the binding of the polypeptide construct to the serum albumin is pH-independent.

In certain embodiments, the polypeptide construct is capable of specifically binding to the serum albumin in the pH range of 5-8 (e.g., 5.5-7.4).

### Fusion protein

In another aspect, the present application also provides a fusion protein, which comprises the nanobody or antigen-binding fragment thereof as described above or the polypeptide construct as described above, and an additional peptide domain.

In certain embodiments, the additional peptide domain is selected from the group consisting of a polypeptide domain with a therapeutic effect.

In certain embodiments, the additional polypeptide domain is connected to the N-terminal and/or C-terminal of the nanobody or antigen-binding fragment thereof or the polypeptide construct, optionally via a peptide linker.

### Isolated nucleic acid molecule

In another aspect, the present application also provides an isolated nucleic acid molecule, which encodes the nanobody or antigen-binding fragment thereof as described above, the polypeptide construct as described above, or the fusion protein as described above.

### Vector

In another aspect, the present application also provides a vector, which comprises the nucleic acid molecule as described above. In certain embodiments, the vector is a cloning vector or an expression vector.

### Host cell

In another aspect, the present application also provides a host cell, which comprises the nucleic acid molecule as described above or the vector as described above. Such host cells include, but are not limited to, prokaryotic cells such as bacterial cells (e.g., *E. coli* cells), and eukaryotic cells such as fungal cells (e.g., yeast cells), insect cells, plant cells and animal cells (e.g., mammalian cells, such as mouse cells, human cells, etc.).

### Preparation method

The nanobody or polypeptide construct or fusion protein of the present application can be prepared by various methods known in the art, such as by genetic engineering recombinant technology. For example, a DNA molecule encoding the nanobody or polypeptide construct or fusion protein of the present application is obtained by chemical synthesis or PCR amplification. The obtained DNA molecule is inserted into an expression vector and then transfected into a host cell. Then, the transfected host cell is cultured under certain conditions and expresses the nanobody or polypeptide construct or fusion protein of the present application.

The antigen-binding fragments of the present application can be obtained by hydrolyzing an intact nanobody molecule (see, Morimoto et al., J. Biochem. Biophys. Methods 24:107-117 (1992) and Brennan et al., Science 229:81 (1985)). In addition, these antigen-binding fragments can also be produced directly by recombinant host cells (reviewed in Hudson, Curr. Opin. Immunol. 11: 548-557 (1999); Little et al., Immunol. Today, 21: 364-370 (2000)). Ordinary technicians in this field are fully aware of other techniques for preparing these antigen-binding fragments.

In another aspect, the present application also provides a method for preparing the nanobody or antigen-binding fragment thereof as described above, the polypeptide construct as described above, or the fusion protein as described above, which comprises culturing the host cell as described above under a condition that allows protein expression, and recovering the nanobody or antigen-binding fragment thereof or the polypeptide construct or the fusion protein from a culture of the cultured host cell.

### Bispecific or multispecific antibody

In another aspect, the present application also provides a bispecific or multispecific antibody, which comprises the nanobody or antigen-binding fragment thereof as described above or the polypeptide construct as described above.

In certain embodiments, the bispecific or multispecific antibody is capable of specifically binding to serum albumin and additionally specifically binding to one or more other targets.

In certain embodiments, the bispecific or multispecific antibody further comprises at least one second antibody or antigen-binding fragment thereof having a binding specificity for a second target.

In certain embodiments, the bispecific or multispecific antibody comprises:
(i) the nanobody or antigen-binding fragment thereof as described above or the polypeptide construct as described above (e.g., a nanobody or antigen-binding fragment thereof or a polypeptide construct containing a sequence as set forth in any one of SEQ ID NOs: 10, 4, 78) as a first antigen-specific binding domain;
(ii) a second antigen-specific binding domain (e.g., a Fab) comprising a light chain as set forth in SEQ ID NO: 79 and a heavy chain as set forth in SEQ ID NO: 80;
   and,
(iii) a third antigen-specific binding domain (e.g., a nanobody VHH) as set forth in SEQ ID NO: 81.

In certain embodiments, the bispecific or multispecific antibody comprises a first polypeptide chain and a second polypeptide chain, wherein the first polypeptide chain comprises (e.g., from N-terminal to C-terminal): a light chain or heavy chain (e.g., VH and CH1) of the second antigen-specific binding domain, the third antigen-specific binding domain, the first antigen-specific binding domain; and, the second polypeptide chain comprises (e.g., from N-terminal to C-terminal): a heavy chain (e.g., VH and CH1) or light chain of the second antigen-specific binding domain, the third antigen-specific binding domain; wherein the first polypeptide chain is paired with the second polypeptide chain to form a complete second antigen-specific binding domain. In certain embodiments, each domain in the first polypeptide chain and/or the second polypeptide chain is connected by a linker (e.g., a peptide linker comprising one or more glycines and/or one or more serines).

In certain embodiments, the bispecific or multispecific antibody comprises:
(1) a first peptide having a sequence as set forth in SEQ ID NO: 69, and a second peptide having a sequence as set forth in SEQ ID NO: 76;
(2) a first peptide having a sequence as set forth in SEQ ID NO: 70, and a second peptide having a sequence as set forth in SEQ ID NO: 76;
   or,
(3) a first peptide having a sequence as set forth in SEQ ID NO: 71, and a second peptide having a sequence as set forth in SEQ ID NO: 76.

In certain embodiments, the first peptide is present in a first peptide chain, and the second peptide is present in a second peptide chain. In certain embodiments, the first peptide chain and the second peptide chain form a complex.

### Conjugate

In another aspect, the present application also provides a conjugate, which comprises the nanobody or antigen-binding fragment thereof as described above or the polypeptide construct as described above, and a therapeutic agent linked to the nanobody or antigen-binding fragment thereof or the polypeptide construct.

In certain embodiments, the therapeutic agent is selected from the group consisting of anti-tumor drugs, such as cytotoxic agent, hormone drug, biological response modifier, another antibody or antigen-binding fragment thereof.

### Pharmaceutical composition

In another aspect, the present application also provides a pharmaceutical composition, which comprises the nanobody or antigen-binding fragment thereof, polypeptide construct, fusion protein, isolated nucleic acid molecule, vector, host cell, bispecific or multispecific antibody or conjugate as described above.

In certain embodiments, the pharmaceutical composition further comprises a pharmaceutically acceptable carrier and/or excipient.

In certain exemplary embodiments, the pharmaceutically acceptable carrier and/or excipient comprises a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), surfactant-containing solution (e.g., a solution containing 0.01% polysorbate 20), pH buffer solution (e.g., phosphate buffer solution), Ringer's solution, and any combination thereof.

In certain embodiments, the pharmaceutical composition comprises the nanobody or antigen-binding fragment thereof or polypeptide construct as described above, or a nucleic acid molecule, vector or host cell encoding the nanobody or antigen-binding fragment thereof or polypeptide construct.

In certain embodiments, the pharmaceutical composition comprises the fusion protein as described above, or a nucleic acid molecule, vector or host cell encoding the fusion protein.

In certain embodiments, the pharmaceutical composition comprises the bispecific or multispecific antibody as described above, or a nucleic acid molecule, vector or host cell encoding the bispecific or multispecific antibody.

In certain embodiments, the pharmaceutical composition comprises a conjugate as described above.

### Use

In another aspect, the present application also provides a use of the nanobody or antigen-binding fragment thereof, polypeptide construct, fusion protein, isolated nucleic acid molecule, vector, host cell, bispecific or multispecific antibody or conjugate, or pharmaceutical composition as described above in the manufacture of a medicament.

In certain embodiments, the medicament can directly or indirectly participate in FcRn-mediated serum albumin circulation in a subject.

In certain embodiments, the medicament shows an prolongated in vivo half-life relative to a corresponding drug lacking the nanobody or antigen-binding fragment thereof.

In certain embodiments, the medicament is a protein drug.

In certain embodiments, the subject is a mammal, such as a human, a mouse or a cynomolgus monkey.

### Method for prolonging in vivo half-life of drug

In another aspect, the present application also provides a method for prolonging in vivo half-life of a drug, comprising: linking the nanobody or antigen-binding fragment thereof as described above or the polypeptide construct as described above to the drug.

In certain embodiments, the prolongation of in vivo half-life is relative to the in vivo half-life of the drug in the absence of the nanobody or antigen-binding fragment thereof.

In certain embodiments, the drug is selected from the group consisting of macromolecular drugs (e.g., polypeptide drugs).

In certain embodiments, the subject is a mammal, such as a human, a mouse or a cynomolgus monkey.

### Definition of terms

In the present application, unless otherwise specified, the scientific and technical terms used herein have the meanings commonly understood by those skilled in the art. In addition, the virology, biochemistry, and immunology laboratory operation steps used herein are all routine steps widely used in the corresponding fields. At the same time, in order to better understand the present application, the definitions and explanations of the relevant terms are provided below.

When the terms "for example", "e.g.", "such as", "including", "comprising" or variants thereof are used herein, these terms will not be considered as restrictive terms, but will be interpreted as meaning "but not limited to" or "not limited to".

Unless otherwise indicated herein or clearly contradicted by the context, the terms "a" and "an" and "the" and similar designations should be interpreted as covering both the singular and the plural in the context of describing the present application (especially in the context of the following claims).

As used herein, the term "nanobody" has the meaning commonly understood by those skilled in the art, which refers to an antibody fragment consisting of a single monomeric variable antibody domain (e.g., a single heavy chain variable region), usually derived from a variable region of a heavy chain antibody (e.g., a camelid antibody or a shark antibody). Typically, a nanobody consists of 4 framework regions and 3 complementarity-determining regions, with a structure of FR1-CDR1-FR2-CDR2-FR3-CDR3-FR4. A nanobody may be truncated at the N-terminal or C-terminal so that it contains only part of FR1 and/or FR4, or lacks one or two of those framework regions, as long as it substantially maintains the antigen binding ability and specificity. Nanobody is also called single-domain antibody (sdAb), and the two can be used interchangeably.

As used herein, the term "antigen-binding fragment" of a nanobody refers to a polypeptide comprising a fragment of a nanobody, which retains the ability to specifically bind to the same antigen to which the nanobody binds, and/or competes with the nanobody for specific binding to the antigen, and which is also referred to as an "antigen-binding portion". See generally, Fundamental Immunology, Ch. 7 (Paul, W., ed., 2nd edition, Raven Press, N.Y. (1989), which is incorporated herein by reference in its entirety for all purposes. An antigen-binding fragment of the nanobody of the present application can be produced by recombinant DNA technology or by enzymatic or chemical cleavage of the nanobody of the present application. In some embodiments, the "antigen-binding fragment" of nanobody can be truncated at the N-terminal or C-terminal so that it only contains part of FR1 and/or FR4, or lacks one or two of those framework regions as compared to the full-length nanobody, as long as it substantially retains the antigen binding ability and specificity.

Antigen-binding fragments of nanobodies can be obtained from a given nanobody (e.g., the nanobody provided by the present application) using conventional techniques known to those skilled in the art (e.g., recombinant DNA technology or enzymatic or chemical cleavage methods), and the antigen-binding fragments of nanobodies are screened for specificity in the same manner as for the intact nanobody.

As used herein, unless the context clearly indicates otherwise, when the term "nanobody" is mentioned, it includes not only the intact nanobody, but also antigen-binding fragments of the nanobody.

As used herein, the term "complementarity-determining region" or "CDR" refers to those amino acid residues in a variable region of an antibody that are responsible for antigen binding. There are three CDRs in nanobody, named CDR1, CDR2 and CDR3. The precise boundaries of these CDRs may be defined according to various numbering systems known in the art, for example, according to the definitions in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883) or the IMGT numbering system (Lefranc et al., Dev. Comparat. Immunol. 27:55-77, 2003). For a given Nanobody, a person skilled in the art will readily identify the CDRs defined by each numbering system. Moreover, the correspondence between different numbering systems is well known to those skilled in the art (for example, see Lefranc et al., Dev. Comparat. Immunol. 27:55-77, 2003). As used herein, the CDRs of a nanobody are preferably determined by the IMGT numbering system.

As used herein, the term "framework region" or "FR" residues refers to those amino acid residues in an antibody variable region other than the CDR residues defined above.

As used herein, the term "Fc domain" or "Fc region" refers to a portion of heavy chain constant region containing CH2 and CH3. The Fc fragment of an antibody has a variety of different functions, but does not participate in antigen binding. "Effector functions" mediated by the Fc region include Fc receptor binding; Clq binding and complement dependent cytotoxicity (CDC); antibody-dependent cell-mediated cytotoxicity (ADCC); phagocytosis; downregulation of cell surface receptors (e.g., B cell receptors); and B cell activation, etc. In some embodiments, the Fc region comprises a hinge, CH2 and CH3. When the Fc region comprises a hinge, the hinge mediates dimerization between two Fc-containing polypeptides. The Fc region can be any isotype of antibody heavy chain constant region, such as IgG1, IgG2, IgG3, or IgG4.

The Fc domain may be a natural Fc region or a variant Fc region. A natural Fc region comprises an amino acid sequence that is consistent with the amino acid sequence of an Fc region found in the nature, for example a human Fc region with a natural sequence comprises a human IgG1 (both non-A and A subtypes) Fc region with a natural sequence; a human IgG2 Fc region with a natural sequence; a human IgG3 Fc region with a natural sequence; and a human IgG4 Fc region with a natural sequence, and naturally occurring variants thereof. A variant Fc region comprises an amino acid sequence that differs from the amino acid sequence of a Fc region with a natural sequence due to at least one amino acid modification. In some embodiments, a variant Fc region can have an altered effector function (e.g., Fc receptor binding, antibody glycosylation, number of cysteine residues, effector cell function, or complement function) as compared to a natural Fc region.

As used herein, the term "humanized antibody" refers to a non-human antibody that has been genetically engineered, and its amino acid sequence has been modified to improve the homology with the sequence of a human antibody. Generally speaking, all or part of the CDR regions of a humanized antibody come from a non-human antibody (donor antibody), and all or part of the non-CDR regions (e.g., variable region FR and/or constant region) come from a human immunoglobulin (receptor antibody). In certain embodiments, the CDR regions of a humanized antibody come from a non-human antibody (donor antibody), and all or part of the non-CDR regions (e.g., variable region FR and/or constant region) come from a human immunoglobulin (receptor antibody). Humanized antibodies generally retain the expected properties of donor antibodies, including but not limited to, antigen specificity, affinity, reactivity, etc. In the present application, the donor antibody may be a camel-derived antibody with expected properties (e.g., antigen specificity, affinity, reactivity, etc.). To prepare a humanized antibody, the CDR regions of an antibody from an immunized animal may be inserted into a human framework sequence using methods known in the art. In the context of nanobody, humanized antibody may refer to humanized VHH, i.e., a VHH in which one or more framework regions have been substantially replaced by human framework regions. In some cases, certain framework regions (FRs) of human immunoglobulin are replaced by corresponding non-human residues. In addition, a humanized VHH may contain residues that are not found in either the initial VHH or human framework sequences, but are included to further improve and optimize the performance of the VHH or VHH-containing polypeptide.

As used herein, the term "identity" is used to refer to the matching of sequences between two polypeptides or between two nucleic acids. In order to determine the percent identity of two amino acid sequences or two nucleic acid sequences, the sequences are aligned for optimal comparison purposes (e.g., a gap may be introduced in the first amino acid sequence or nucleic acid sequence for optimal alignment with the second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percent identity between two sequences is a function of the number of identical positions shared by the sequences (i.e., percent identity = number of identical overlapping positions/total number of positions x 100%). In certain embodiments, the two sequences are of the same length.

The determination of percent identity between two sequences can also be achieved using a mathematical algorithm. A non-limiting example of a mathematical algorithm for comparison of two sequences is the algorithm of Karlin and Altschul, 1990, Proc. Natl. Acad. Sci. U.S.A. 87:2264-2268, as improved in Karlin and Altschul, 1993, Proc. Natl. Acad. Sci. U.S.A. 90:5873-5877. Such algorithms are integrated into the NBLAST and XBLAST programs of Altschul et al., 1990, J. Mol. Biol. 215:403.

As used herein, the term "specific binding" refers to a non-random binding reaction between two molecules, such as a reaction between an antibody and an antigen to which it is directed. The strength or affinity of a specific binding interaction can be expressed by the equilibrium dissociation constant (K_{D}) of the interaction. In the present application, the term "K_{D}" refers to the dissociation equilibrium constant of a specific antibody-antigen interaction, which is used to describe the binding affinity between the antibody and the antigen. The smaller the equilibrium dissociation constant, the tighter the antibody-antigen binding, and the higher the affinity between the antibody and the antigen.

The specific binding properties between two molecules can be determined using methods known in the art. One method involves measuring the rate of formation and dissociation of antigen binding site/antigen complex. Both "association rate constant" (kₐ or kₒₙ) and "dissociation rate constant" (k_{dis} or k_{off}) can be calculated from concentrations and actual rates of association and dissociation (see Malmqvist M, Nature, 1993, 361: 186-187). The ratio of k_{dis}/kₒₙ is equal to the dissociation constant K_{D} (see Davies et al., Annual Rev Biochem, 1990; 59:439-473). K_{D}, kₒₙ and k_{dis} values can be measured by any effective method. In certain embodiments, the dissociation constant can be measured by surface plasmon resonance (SPR) with a Biacore instrument. In addition, the dissociation constant can be measured by bioluminescence interferometry or Kinexa.

As used herein, the term "vector" refers to a nucleic acid carrier into which a polynucleotide can be inserted. When the vector can express the protein encoded by the inserted polynucleotide, the vector is called an expression vector. The vector can be introduced into a host cell by transformation, transduction or transfection so that the genetic material elements it carries are expressed in the host cell. Vectors are well known to those skilled in the art, and include but are not limited to: plasmid; phagemid; cosmid; artificial chromosome, such as yeast artificial chromosome (YAC), bacterial artificial chromosome (BAC) or P1-derived artificial chromosome (PAC); bacteriophage such as lambda phage or M13 phage and animal virus, etc. Animal viruses that can be used as vectors include, but are not limited to, retrovirus (including lentivirus), adenovirus, adeno-associated virus, herpes virus (e.g., herpes simplex virus), poxvirus, baculovirus, papillomavirus, papovavirus (e.g., SV40). A vector can contain a variety of elements that control expression, including, but not limited to, promoter sequence, transcription start sequence, enhancer sequence, selection element, and reporter gene. In addition, the vector may also contain a replication origin.

As used herein, the term "host cell" refers to a cell that can be used to introduce vector, including, but not limited to, prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell, or human cell.

As used herein, the term "conservative substitution" refers to an amino acid substitution that does not adversely affect or change the expected properties of the protein/polypeptide containing the amino acid sequence. For example, conservative substitutions can be introduced by standard techniques known in the art, such as site-directed mutagenesis and PCR-mediated mutagenesis. Conservative amino acid substitutions include substitutions of amino acid residues with amino acid residues having similar side chains, such as substitutions with residues physically or functionally similar to the corresponding amino acid residues (e.g., having similar size, shape, charge, chemical properties, including the ability to form covalent bond or hydrogen bond, etc.). Families of amino acid residues with similar side chains have been defined in the art. These families include amino acids with basic side chains (e.g., lysine, arginine and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

The compilation of the twenty conventional amino acids involved herein follows conventional usage. See, for example, Immunology-A Synthesis (2nd Edition, E. S. Golub and D. R. Gren, Eds., Sinauer Associates, Sunderland, Mass. (1991)), which is incorporated herein by reference. In the present application, the terms "polypeptide" and "protein" have the same meaning and are used interchangeably. And in the present application, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995), and includes but is not limited to: pH regulator, surfactant, adjuvant, ionic strength enhancer, diluent, agent for maintaining osmotic pressure, agent for delaying absorption, preservative. For example, the pH regulator includes but is not limited to phosphate buffer. The surfactant includes but is not limited to cationic, anionic or non-ionic surfactant, such as Tween-80. The ionic strength enhancer includes but is not limited to sodium chloride. The preservative includes but is not limited to various antibacterial agent and antifungal agent, such as paraben, chlorobutanol, phenol, sorbic acid, etc. The agent for maintaining osmotic pressure includes, but is not limited to, sugar, NaCl and the like. The agent for delaying absorption includes, but is not limited to, monostearate and gelatin. The diluent includes, but is not limited to, water, aqueous buffer (e.g., buffered saline), alcohol and polyol (e.g., glycerol), etc. The preservative includes, but is not limited to, various antibacterial and antifungal agents, such as thimerosal, 2-phenoxyethanol, paraben, chlorobutanol, phenol, sorbic acid, etc. Stabilizers have the meaning commonly understood by those skilled in the art, which can stabilize the desired activity of the active ingredient in the drug, including, but not limited to, sodium glutamate, gelatin, SPGA, sugar (e.g., sorbitol, mannitol, starch, sucrose, lactose, dextran, or glucose), amino acid (e.g., glutamic acid, glycine), protein (e.g., dried whey, albumin or casein) or degradation product thereof (e.g., lactalbumin hydrolysate), etc. In certain exemplary embodiments, the pharmaceutically acceptable carrier or excipient contains a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), surfactant-containing solution (e.g., a solution containing 0.01% polysorbate 20), pH buffer solution (e.g., phosphate buffer solution), Ringer's solution and any combination thereof.

As used herein, the term "in vivo half-life" has a meaning known to those skilled in the art, and can generally be defined as the time required for the serum concentration of a molecule in vivo to decrease by 50% (e.g., due to degradation of ligand and/or clearance or chelation of ligand through natural mechanisms). Methods for determining the in vivo half-life are well known to those skilled in the art, for example, by pharmacokinetic analysis.

### Beneficial effects of the present application

The present application provides a nanobody having high binding activity to serum albumin, which has cross-reactivity with human, monkey and/or mouse serum albumin. In particular, the binding of the nanobody of the present application to serum albumin is pH-independent, and it can effectively participate in the FcRn-mediated serum albumin circulation, thereby extending the in vivo half-life of the nanobody. Therefore, the nanobody of the present application can be coupled (e.g., fused) with a drug molecule (e.g., a polypeptide drug) to extend the half-life of the drug molecule in vivo.

In addition, the nanobody also has the characteristics of small molecular weight and good stability. Compared with traditional common antibodies, it has many advantages in drug development, such as good tissue infiltration, flexible administration, high degree of humanization, and facile transformation into recombinant protein.

The following will describe the embodiments of the present application in detail with reference to the drawings and examples, but those skilled in the art will understand that the following drawings and examples are only used to illustrate the present application, rather than to limit the scope of the present application. According to the following detailed description of the drawings and preferred embodiments, the various objects and advantages of the present application will become apparent to those skilled in the art.

### Brief Description of the Drawings

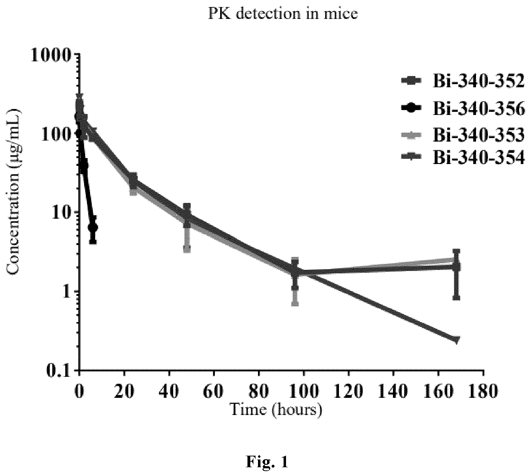

Fig. 1 shows the changes in the concentrations of Bi-340-352, Bi-340-353, Bi-340-354 and Bi-340-356 in mouse serum over time.

### Specific Models for Carrying Out the present invention

The present invention is now described with reference to the following examples intended to illustrate the present invention (but not to limit the present invention).

Unless otherwise specified, the molecular biology experimental methods and immunoassays used in the present invention were basically carried out in accordance with the methods described in J. Sambrook et al., Molecular Cloning: A Laboratory Manual, 2nd edition, Cold Spring Harbor Laboratory Press, 1989, and F. M. Ausubel et al., A Compendium of Molecular Biology Experimental Guide, 3rd edition, John Wiley & Sons, Inc., 1995; the use of restriction endonucleases was in accordance with the conditions recommended by the product manufacturer. It is known to those skilled in the art that the examples describe the present invention by way of example and are not intended to limit the scope of the present invention as claimed.

### Example 1: Screening of anti-HSA nanobody

### 1.1 Construction of nanobody library

### Animal immunization

1 mg of human serum albumin (HSA) antigen (purchased from AcroBiosystems) was mixed with an equal volume of Freund's adjuvant, and used to immunize two alpacas, once a week, for a total of 4 immunizations, to stimulate B cells to express antigen-specific nanobodies. After the 4 immunizations, 50 ml of alpaca peripheral blood was extracted, and lymphocytes were separated using lymphocyte separation solution. Total RNA was extracted using RNA extraction reagent Trizol (purchased from Invitrogen). Total cDNA of alpaca lymphocytes was obtained by reverse transcription using a cDNA synthesis kit (purchased from Invitrogen).

### Nanobody gene amplification

The first round of PCR was performed to amplify IgG2 and IgG3 sequences from the cDNA obtained in the previous step. The primers used for amplification were shown in Table 1:

**Table 1. First round PCR primers**

| Name | Sequence number | Sequence (5' to 3') |
|---|---|---|
| Upstream primer | SEQ ID NO: 60 | GTCCTGGCTGCTCTTCTACAAGG |
| Downstream primer | SEQ ID NO: 61 | GGTACGTGCTGTTGAACTGTTCC |

The first round PCR products were subjected to agarose gel electrophoresis, and the desired band at 750 bp was excised from the gel and recovered for the second round amplification for VHH sequence. The second round PCR amplification primers were shown in Table 2:

**Table 2. Second round PCR primers**

| Name | Sequence number | Sequence (5' to 3') |
|---|---|---|
| Upstream primer | SEQ ID NO: 62 | CTAGTGCGGCCGCCTGGAGACGGTGAC CTGGGT |
| Downstream primer | SEQ ID NO: 63 | CGCGGATCCCAGGTGCAGCTGCAGGAG TCTGGRGGAGG |

Using the second round PCR product as a template, the third round PCR was performed to add a homology arm sequence, which is homologous to the display vector, to the VHH gene. The third round PCR amplification primers were shown in Table 3:

**Table 3. Third round PCR primers**

| Name | Sequence number | Sequence (5' to 3') |
|---|---|---|
| Upstream primer | SEQ ID NO: 64 | ATTTTTACTGCTGTTTTATTCGCAGCATC CTCCGCATTAGCTAAAAGAGAGGCTGA AGCACAGGTGCAGCTGCAGGAGTCTGG RGGAGG |
| Downstream primer | SEQ ID NO: 65 | AGTTGTCAGTTCCTGTGCCCCCCCTCCT CCCGCGCCACCTCCGCCCGCACCTCCGC CACCAcTGGAGACGGTGACCTGGGT |
| Upstream homology arm sequence | SEQ ID NO: 66 | ATTTTTACTGCTGTTTTATTCGCAGCATC CTCCGCATTAGCTAAAAGAGAGGCTGA AGCA |
| Downstream homology arm sequence | SEQ ID NO: 67 | AGTTGTCAGTTCCTGTGCCCCCCCTCCT CCCGCGCCACCTCCGCCCGCACCTCCGC CACC |

The target fragment was recovered using a PCR purification kit (purchased from QIAGEN).

### Library construction

The linearized yeast display vector (artificially synthesized) and the third round PCR products were mixed and electrotransformed into *Saccharomyces cerevisiae* (purchased from ATCC) to construct anti-HSA nanobody libraries from two animals, and the library size was measured as 2.1×10⁷ and 3.2×10⁷, respectively.

### 1.2 Screening of HSA nanobodies

### Biotinylation of HSA protein

An appropriate volume of double distilled water was taken to dissolve HSA protein (purchased from AcroBiosystems, catalog number: HSA-H5220), and according to the product instructions of biotin labeling kit (purchased from Thermo), the biotin was dissolved and mixed with the protein solution, and incubated at 4°C for 2 hours. A desalting column (purchased from Thermo) was used to remove excess biotin, and the desalting column pretreatment and sample collection operations were all carried out according to the product instructions.

### MACS enrichment of yeast capable of specifically binding to HSA

The VHH library constructed in step 1.1 was inoculated into SD-CAA expansion medium (1L of SD-CAA expansion medium containing 6.7g of YNB, 5g of tyrosine, 13.62g of Na₂HPO₄·12H₂O, 7.44g of NaH₂PO₄ and 2% glucose) and cultured overnight at 30°C and 225rpm. An appropriate amount of yeast cells was taken, centrifuged at 3000rpm×5min (the parameters of the following centrifugation operation were the same as here) to remove the medium, the yeast cells were resuspended with SD-CAA induction medium, and induced overnight. The concentration of the library after induction was determined, an appropriate amount of yeast cells was taken, centrifuged to remove the medium. The yeast cells were resuspended with 50ml of PBS and centrifuged to remove the supernatant. The yeast cells were resuspended with 10ml of PBS.

The biotin-labeled HSA protein obtained in the above step (the final concentration was 100nM) was added, and incubated at room temperature for 30min; and the yeast cells were collected by centrifugation, and washed 3 times with 50ml of PBS. The yeast cells were resuspended with 5ml of washing solution, added with 200µl of SA magnetic beads (purchased from Miltenyi Biotec), and incubated for 10min. The mixture of the yeast cells and the magnetic beads was washed 3 times with PBS, and added to a LS purification column (purchased from Miltenyi Biotec). The LS purification column was placed on a magnetic grate, and washed with PBS to remove non-specifically bound yeast cells. The column was taken out the magnetic grate, and added with PBS to elute the yeast cells. After centrifugation, the eluted yeast cells were transferred to SD-CAA expansion medium for expansion.

### Flow cytometric sorting to obtain high-affinity yeast cells

The yeast cells enriched by MACS were inoculated into SD-CAA expansion medium, and cultured in a shake flask at 30°C and 225rpm overnight. The yeast cells were resuspended with SD-CAA induction medium and induced overnight. Anti-c-Myc mouse antibody (purchased from Thermo, catalog number: MA1-980) and 100nM biotin-labeled HSA antigen were added and incubated for 10min. The yeast cells were washed 3 times with PBS, then goat anti-mouse IgG (H+L) Alexa Fluor Plus 488 fluorescent antibody (purchased from Invitrogen, catalog number: A32723) and streptavidin-APC conjugate fluorescent antibody (purchased from Invitrogen, catalog number: SA1005) were added, and incubated for 15 minutes. The cells were resuspended with PBS, and sorted by using BD FACSAriaII instrument to obtain yeast cells with high binding ability to HSA antigen.

### Sequencing of the vHH candidates

The yeast output with high binding ability to HSA antigen obtained by MACS and FACS enrichment was cultured overnight at 30°C and 225rpm in SD-CAA expansion medium, and yeast plasmid was extracted according to the operation of yeast plasmid extraction kit (purchased from Tiangen). The plasmid was transformed into Top10 competent cells (purchased from Tiangen) by electroporation, coated on a plate with ampicillin, and cultured overnight at 37°C. The monoclonal cell was picked for sequencing to obtain the VHH sequence, and the CDR sequence was determined according to the IMGT numbering system. The sequence information of the monoclonal nanobody obtained was shown in Table 4.

**Table 4: VHH and CDR sequence information of candidate anti-HSA nanobodies**

| No. | VHH | CDR1 | CDR2 | CDR3 |
|---|---|---|---|---|
| HSA-8 | QVQLQESGGGLVQPGGSLRLSCAAASGFTLDYYAIGWFRQAP GKEREGVSCISSSGGSTNSADSVKGRFTISRDNAKNTVYLQMN SLKPEDTAVYYCAAATLECRTVVVGYDYWGQGTQVTVSS | SGFTLDYYA | ISSSGGST | AAATLECRTVVVG YDY |
| | SEQ ID NO: 1 | SEQ ID NO: 30 | SEQ ID NO: 39 | SEQ ID NO: 47 |
| HSA-30 | QVQLQESGGGLVQPGGSLRLSCKASGFTFSIYSMGWYRQVPG KEREAVATISSGGSPFYANSASGRFTISRDNAKSTVYLQMNNL KPEDTGVYSCSPITSIFKAQGQGTQVTVSS | GFTFSIYS | ISSGGSP | SPITSIFKA |
| | SEQ ID NO: 2 | SEQ ID NO: 31 | SEQ ID NO: 40 | SEQ ID NO: 48 |
| HSA-59 | QVQLQESGGGLVQAGGSLRLSCAASGFTLDYYAIGWFRQAPG KEREEVSCISSSGGSTNYADSVKGRFTISRDNAKNTVYLQMNS LKPEDTGVYYCAAALLECRVRSWPSDNWGQGTQVTVSS | GFTLDYYA | ISSSGGST | AAALLECRVRSWP SDN |
| | SEQ ID NO: 3 | SEQ ID NO: 32 | SEQ ID NO: 41 | SEQ ID NO: 49 |
| HSA-76 | QVQLQESGGGLVQPGGSLRLSCAASGSIFTFYRMAWYRRAPG KERELVALITTDTSTNYADSVKGRFTISRDYAKNTVYLQMDSL KPEDTAAYYCNVRNVERDYWGQGTQVTVSS | GSIFTFYR | ITTDTST | NVRNVERDY |
| | SEQ ID NO: 7 | SEQ ID NO: 33 | SEQ ID NO: 42 | SEQ ID NO: 50 |
| HSA-M-16 | QVQLQESGGGLVQPGGSLRLSCAASGFTLDYYAIGWFRQAPG KEREGVSCIASSGGSTNYADYVKGRFTISRDNAKNTVYLLMN KLKPEDTAVYYCAAAVLECRTVVREYDYWGQGTQVTVSS | GFTLDYYA | IASSGGST | AAAVLECRTVVRE YDY |
| | SEQ ID NO: 8 | SEQ ID NO: 32 | SEQ ID NO: 43 | SEQ ID NO: 51 |
| HSA-M-27 | QVQLQESGGGLVQPGGSLRLSCAASGFTLDYYAIGWFRQAPG KEREGVSCIASSGGSTNYADSVKGRFTISRDNAKNTVYLQMN KLKPEDTAVYYCAAAVLECRTVVRGYDYWGQGTQVTVSS | GFTLDYYA | IASSGGST | AAAVLECRTVVRG YDY |
| | SEQ ID NO: 9 | SEQ ID NO: 32 | SEQ ID NO: 43 | SEQ ID NO: 52 |
| HSA-M-29 | QVQLQESGGGLVQPGGSLRLSCVASRFTLDYYAIGWFRQAPG KEREGVSCIASSGGSTNYADSVKGRFTISRDNAKNTVYLQMN KLKPEDTAVYYCAAAVLECRTVVRGYDYWGQGTQVTVSS | RFTLDYYA | IASSGGST | AAAVLECRTVVRG YDY |
| | SEQ ID NO: 14 | SEQ ID NO: 35 | SEQ ID NO: 43 | SEQ ID NO: 52 |
| HSA-W-5 | QVQLQESGGGLVQPGGSLRLSCAASGFTLDYYAIGWFRQAPG KEREGVSCIASSGGSTNYADSVKGRFTISRDNAKNTVYLQMNS LKPEDTAVYYCAAATLECRTTLTGYAYWGQGTQVTVSS | GFTLDYYA | IASSGGST | AAATLECRTTLTGY AY |
| | SEQ ID NO: 15 | SEQ ID NO: 32 | SEQ ID NO: 43 | SEQ ID NO: 53 |
| HSA-W-7 | QVQLQESGGGLVQPGGSLRLSCEAASGFTLDYYAIGWFRQAP GKEREGVSCISSSGGSTNSADSVKGRFTISRDNAKNTVYLQMN SLKPEDTAVYYCAAATLECRTVVVGYDYWGQGTQVTVSS | SGFTLDYYA | ISSSGGST | AAATLECRTVVVG YDY |
| | SEQ ID NO: 16 | SEQ ID NO: 30 | SEQ ID NO: 39 | SEQ ID NO: 47 |
| HSA-W-14 | QVQLQESGGGLVQPGGSLRLSCTASGSTLDYYAIGWFRQAPG KEREGVSCISSSGGSTIYADSVKGRFTISRDNAKNTVYLQMNSL KPEDTAVYYCAAAVLECRAAEYVNSWGQGTQVTVSS | GSTLDYYA | ISSSGGST | AAAVLECRAAEYV NS |
| | SEQ ID NO: 17 | SEQ ID NO: 36 | SEQ ID NO: 41 | SEQ ID NO: 54 |
| HSA-W-15 | QVQLQESGGGLVQPGGSLRLSCAASRFTLDYYAIAWFRQAPG KDLEEVSCISSSGGATNYADSVKGRFTISRDNAKNTVYLQMNS LKPEETAVYYCAAATLECRGRSSSYDYWGQGTQVTVSS | RFTLDYYA | ISSSGGAT | AAATLECRGRSSSY DY |
| | SEQ ID NO: 18 | SEQ ID NO: 35 | SEQ ID NO: 44 | SEQ ID NO: 55 |
| HSA-W-17 | QVQLQESGGGLVQPGGSLRLSCAASGFTLDYYAIAWFRQAPG KDLEGVSCIASSGGATNYADSVKGRFTISRDNAKNTVYLQMN KLKPEETAVYYCAAATFECRGRSSSYDYWGQGTQVTVSS | GFTLDYYA | IASSGGAT | AAATFECRGRSSSY DY |
| | SEQ ID NO: 19 | SEQ ID NO: 32 | SEQ ID NO: 45 | SEQ ID NO: 56 |
| HSA-W-18 | QVQLQESGGGLVQPGGSLRLSCAASRFTLDYYAIGWFRQAPG KEREGVSCIASSGGSTNYADSVKGRFTISRDNAKNTVYLQMN KLKPEDTAVYYCAAAVLECRTVVRGYDYWGQGTQVTVSS | RFTLDYYA | IASSGGST | AAAVLECRTVVRG YDY |
| | SEQ ID NO: 20 | SEQ ID NO: 35 | SEQ ID NO: 43 | SEQ ID NO: 52 |
| HSA-W-19 | QVQLQESGGGLVQPGGSLRLSCAASGFTLDYYAIGWFRQAPG KEREGVSCISSSGGSTNNADSVKGRFTISRDNAENTVYLQMNS LKPEDTAVYYCAAAVLECRTVVRGYDYWGQGTQVTVSS | GFTLDYYA | ISSSGGST | AAAVLECRTVVRG YDY |
| | SEQ ID NO: 21 | SEQ ID NO: 32 | SEQ ID NO: 41 | SEQ ID NO: 52 |
| HSA-W-20 | QVQLQESGGGLVQPGGSLRLSCAASGFTLDYYAIGWFRQAPG KEREGVSCIASSGGSTNYADSVKGRFTISRDNAENTVYLQMNS LKPEDTAVYYCAAAVLECRTVVRGYDYWGQGTQVTVSS | GFTLDYYA | IASSGGST | AAAVLECRTVVRG YDY |
| | SEQ ID NO: 22 | SEQ ID NO: 32 | SEQ ID NO: 43 | SEQ ID NO: 52 |
| HSA-W-21 | QVQLQESGGGLVQPGGSLRLSCVASGFTLDYYAIGWFRQAPG KEREGVSCISSSGGSTNYADSVKGRFTISRDNAKNTVYLQMNS LKPEDTAVYYCAAAVLECRTVVRGYDYWGQGTQVTVSS | GFTLDYYA | ISSSGGST | AAAVLECRTVVRG YDY |
| | SEQ ID NO: 23 | SEQ ID NO: 32 | SEQ ID NO: 41 | SEQ ID NO: 52 |
| HSA-PH-8 | QVQLQESGGGLVQPGGSLRLSCVASGSIWGIYHMGWYRQAPG KQRELVASITVDGSTHYADTVKGRFTISKDNAANMVYLQMN ALEPEDTAVYYCDIRNVGGDYWGQGTQVTVSS | GSIWGIYH | ITVDGST | DIRNVGGDY |
| | SEQ ID NO: 24 | SEQ ID NO: 37 | SEQ ID NO: 46 | SEQ ID NO: 57 |
| HSA-PH-10 | QVQLQESGGGLVQPGGSLRLSCAASGFTLDYYAIGWFRQAPG KEREGVSCISSSGGSTNYADSVKGRFTISKDNAKNTVYLQMNS LKPEDTAVYYCAAATLECRTVIRGYDYWGQGTQVTVSS | GFTLDYYA | ISSSGGST | AAATLECRTVIRGY DY |
| | SEQ ID NO: 28 | SEQ ID NO: 32 | SEQ ID NO: 41 | SEQ ID NO: 58 |
| HSA-PH-11 | QVQLQESGGGLVQPGGSLRLSCTASGAQLEYYAIGWFRQAPG KEREGVSCISSSGGSTNNADSVKGRFTISRDNAKNTVYLQMNS LKPEDTAVYYCAAATLECRTVLTGYDYWGQGTQVTVSS | GAQLEYYA | ISSSGGST | AAATLECRTVLTG YDY |
| | SEQ ID NO: 29 | SEQ ID NO: 38 | SEQ ID NO: 41 | SEQ ID NO: 59 |

### Example 2. Construction, expression and purification of heavy chain antibody

### 2.1. Construction of a pCDNA3.1 expression vector inserted with the antibody gene

VHH gene sequence was ligated to the coding sequence of human IgG1 (LALA mutation) Fc fragment (the amino acid sequence was set forth in SEQ ID NO: 68), and inserted into pCDNA3.1 vector that was linearized by *EcoR* I/*Not* I double enzyme digestion, using the recombinase that catalyzes homologous recombination (purchased from Vazyme, catalog number: c115-02), in which the process followed the product manual. The homologous recombination product was transferred into Top10 competent cells, coated on a plate with ampicillin, and cultured at 37°C overnight. Single clones were picked for sequencing, and plasmids were extracted.

The sequence of SEQ ID NO: 68 was as follows:

DKTHTCPPCPAPEAAGGPSVFLFPPKPKDTLMISRTPEVTCVVVDVSHE
DPEVKFNWYVDGVEVHNAKTKPREEQYNSTYRVVSVLTVLHQDWLNGKEYKCKVSN
KALPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSLSL
SPG

### 2.2. Cell transfection and protein purification

The extracted plasmid obtained in the above steps was transferred into Expi-CHO cells by using ExpiCHO^{™} expression system kit (purchased from Thermo). The transfection method was carried out according to the product manual. After 5 days of cell culture, the supernatant was collected, and the protein was purified using protein A magnetic beads (purchased from GenScript) via a sorting method. The magnetic beads were resuspended in an appropriate volume (1-4 times the volume of the magnetic beads) of binding buffer (PBS + 0.1% Tween 20, pH 7.4), then added to the sample to be purified, and incubated at room temperature for 1 hour with gentle shaking. The sample was placed on a magnetic grate (purchased from Beaver), the supernatant was discarded, and the magnetic beads were washed three times with binding buffer. Elution buffer (0.1M sodium citrate, pH 3.2) in a volume 3-5 times the volume of the magnetic beads was added, and shaken at room temperature for 5-10 minutes. After placing back on the magnetic grate, the elution buffer was collected, transferred to a collection tube with neutralization buffer (1M Tris, pH 8.54) and mixed well, thereby obtaining the target protein.

### Example 3. Affinity determination

ForteBio affinity determination was carried out according to the existing method (Estep, P et al., Determination of antibody-antigen affinity and epitope binding based on high-throughput methods. MAbs, 2013.5(2):p.270-8). Briefly, the sensor was equilibrated offline in the assay buffer for 30 min, then tested online for 60 s to establish a baseline, and the purified antibody obtained as described above was loaded online onto the AHQ sensor. The sensor was then exposed to 100 nM HSA antigen (purchased from AcroBiosystems, catalog number: HSA-H5220) for 5 min, and then the sensor was transferred to PBS for dissociation for 5 min. The kinetic analysis was performed using a 1:1 binding model. The affinity data were shown in Table 5.

**Table 5. Affinity of candidate molecules to HSA**

| No. | K_{D}(M) | Kon(1/Ms) | Koff(1/s) |
|---|---|---|---|
| HSA-30 | 1.54E-09 | 1.40E+05 | 2.15E-04 |
| HSA-59 | 2.91E-08 | 5.55E+04 | 1.62E-03 |
| HSA-76 | 7.97E-07 | 8.73E+03 | 6.96E-03 |
| HSA-M-16 | 3.81E-08 | 1.65E+05 | 6.28E-03 |
| HSA-M-27 | 1.61E-08 | 2.44E+05 | 3.93E-03 |
| HSA-M-29 | 2.33E-08 | 2.51E+05 | 5.85E-03 |
| HSA-W-20 | 2.24E-08 | 1.93E+05 | 4.31E-03 |
| HSA-PH-8 | 2.152E-08 | 1.23E+05 | 2.65E-03 |
| HSA-PH-11 | 4.43E-08 | 2.21E+05 | 9.79E-03 |

In this example, the cross-reactions of the candidate molecules with serum albumins of different species were further detected, in which the serum albumins included mouse serum albumin (MSA, purchased from AcroBiosystems, catalog number: MSA-M52H8), and cynomolgus serum albumin (CSA, purchased from AcroBiosystems, catalog number: CSA-C52H4). The affinity data were shown in Table 6.

**Table 6. Affinity of candidate molecules with MSA/CSA**

| No. | | MSA | CSA |
|---|---|---|---|
| HSA-30 | KD(M) | 2.584E-08 | 1.202E-09 |
| | Kon(1/Ms) | 1.37E+05 | 3.25E+05 |
| | Koff(1/s) | 3.54E-03 | 3.90E-04 |
| HSA-59 | KD(M) | 3.107E-08 | 7.732E-08 |
| | Kon(1/Ms) | 1.09E+05 | 1.43E+05 |
| | Koff(1/s) | 3 .40E-03 | 1.11E-02 |
| HSA-76 | KD(M) | 3.134E-08 | 2.567E-08 |
| | Kon(1/Ms) | 1.30E+05 | 2.14E+05 |
| | Koff(1/s) | 4.07E-03 | 5.49E-03 |
| HSA-M-16 | KD(M) | 1.22E-08 | 7.07E-08 |
| | Kon(1/Ms) | 4.47E+05 | 2.84E+05 |
| | Koff(1/s) | 5.46E-03 | 2.01E-02 |
| HSA-M-27 | KD(M) | 1.74E-08 | 7.16E-08 |
| | Kon(1/Ms) | 4.33E+05 | 2.80E+05 |
| | Koff(1/s) | 7.52E-03 | 2.01E-02 |
| HSA-M-29 | KD(M) | 1.77E-08 | 7.43E-08 |
| | Kon(1/Ms) | 4.08E+05 | 2.74E+05 |
| | Koff(1/s) | 7.22E-03 | 2.04E-02 |
| HSA-W-20 | KD(M) | 4.39E-09 | 1.66E-08 |
| | Kon(1/Ms) | 5.40E+05 | 4.73E+05 |
| | Koff(1/s) | 2.37E-03 | 7.85E-03 |
| HSA-PH-8 | KD(M) | 1.05E-08 | 6.64E-09 |
| | Kon(1/Ms) | 2.74E+05 | 2.71E+05 |
| | Koff(1/s) | 2.88E-03 | 1.80E-03 |
| HSA-PH-11 | KD(M) | 6.32E-09 | 9.07E-09 |
| | Kon(1/Ms) | 4.68E+05 | 5.91E+05 |
| | Koff(1/s) | 2.96E-03 | 5.37E-03 |

### Example 4. Construction of humanized anti-HSA antibody

In order to reduce the immunogenicity of monoclonal antibodies in the human body, three antibodies, HSA-59, HSA-M-27 and HSA-PH-8, were humanized. The humanization method adopted the VHH humanized universal framework transplantation method, and at the same time, some amino acids of the antibody framework 2 were mutated according to the method reported in the literature (Vincke, C., et al., General strategy to humanize a camelid single-domain antibody and identification of a universal humanized nanobody scaffold. J Biol Chem 284(5): 3273-3284). The sequence information of the humanized antibodies was shown in Table 7.

**Table 7. VHH and CDR sequence information of humanized antibodies**

| No. | VHH | CDR1 | CDR2 | CDR3 |
|---|---|---|---|---|
| Hu-HSA-59-1 | EVQLLESGGGLVQPGGSLRLSCAASGFTLDYYAIGWFRQAP GKEREEVSCISSSGGSTNYADSVKGRFTISRDNSKNTVYLQM NSLKPEDTAVYYCAAALLECRVRSWPSDNWGQGTQVTVSS | GFTLDYYA | ISSSGGST | AAALLECRVRSW PSDN |
| | SEQ ID NO: 4 | SEQ ID NO: 32 | SEQ ID NO: 41 | SEQ ID NO: 49 |
| Hu-HSA-59-2 | EVQLLESGGGLVQPGGSLRLSCAASGFTLDYYAIGWFRQAP GKGLEEVSCISSSGGSTNYADSVKGRFTISRDNSKNTVYLQM NSLKPEDTAVYYCAAALLECRVRSWPSDNWGQGTQVTVSS | GFTLDYYA | ISSSGGST | AAALLECRVRSW PSDN |
| | SEQ ID NO: 5 | SEQ ID NO: 32 | SEQ ID NO: 41 | SEQ ID NO: 49 |
| Hu-HSA-59-3 | EVQLLESGGGLVQPGGSLRLSCAASGFTLDYYAIGWFRQAP GKEREEVSCISSSGGSTNYADSVKGRFTISRDNSKNTVYLQM NSLRAEDTAVYYCAAALLECRVRSWPSDNWGQGTQVTVSS | GFTLDYYA | ISSSGGST | AAALLECRVRSW PSDN |
| | SEQ ID NO: 6 | SEQ ID NO: 32 | SEQ ID NO: 41 | SEQ ID NO: 49 |
| Hu-HSA-M-27-1 | EVQLLESGGGLVQPGGSLRLSCAASGFTLDYYAIGWFRQAP GKEREGVSCIASSGGSTNYADSVKGRFTISRDNSKNTVYLQM NSLKPEDTAVYYCAAAVLECRTVVRGYDYWGQGTQVTVSS | GFTLDYYA | IASSGGST | AAAVLECRTVVR GYDY |
| | SEQ ID NO: 10 | SEQ ID NO: 32 | SEQ ID NO: 43 | SEQ ID NO: 52 |
| Hu-HSA-M-27-1-2 | EVQLLESGGGLVQPGGSLRLSCAASGETLDYYAIGWFRQAP GKEREGVSCIASSGGSTNYADSVKGRFTISRDNSKNTVYLQM NSLKPEDTAVYYCAAAVLECRTVVRGYDYWGQGTQVTVSS | GETLDYYA | IASSGGST | AAAVLECRTVVR GYDY |
| | SEQ ID NO: 11 | SEQ ID NO: 34 | SEQ ID NO: 43 | SEQ ID NO: 52 |
| Hu-HSA-M-27-2 | EVQLLESGGGLVQPGGSLRLSCAASGFTLDYYAIGWFRQAP GKGLEGVSCIASSGGSTNYADSVKGRFTISRDNSKNTVYLQM NSLKPEDTAVYYCAAAVLECRTVVRGYDYWGQGTQVTVSS | GFTLDYYA | IASSGGST | AAAVLECRTVVR GYDY |
| | SEQ ID NO: 12 | SEQ ID NO: 32 | SEQ ID NO: 43 | SEQ ID NO: 52 |
| Hu-HSA-M-27-3 | EVQLLESGGGLVQPGGSLRLSCAASGFTLDYYAIGWFRQAP GKEREGVSCIASSGGSTNYADSVKGRFTISRDNSKNTVYLQM NSLRAEDTAVYYCAAAVLECRTVVRGYDYWGQGTQVTVSS | GFTLDYYA | IASSGGST | AAAVLECRTVVR GYDY |
| | SEQ ID NO: 13 | SEQ ID NO: 32 | SEQ ID NO: 43 | SEQ ID NO: 52 |
| Hu-HSA-PH-8-1 | QVQLVESGGGLVQPGGSLRLSCSASGSIWGIYHMGWYRQAP GKQRELVASITVDGSTHYADTVKGRFTISRDNSKNMVYLQM NSLEPEDTAVYYCDIRNVGGDYWGQGTQVTVSS | GSIWGIYH | ITVDGST | DIRNVGGDY |
| | SEQ ID NO: 25 | SEQ ID NO: 37 | SEQ ID NO: 46 | SEQ ID NO: 57 |
| Hu-HSA-PH-8-2 | QVQLVESGGGLVQPGGSLRLSCSASGSIWGIYHMGWYRQAP GKGLELVASITVDGSTHYADTVKGRFTISRDNSKNMVYLQM NSLEPEDTAVYYCDIRNVGGDYWGQGTQVTVSS | GSIWGIYH | ITVDGST | DIRNVGGDY |
| | SEQ ID NO: 26 | SEQ ID NO: 37 | SEQ ID NO: 46 | SEQ ID NO: 57 |
| Hu-HSA-PH-8-3 | QVQLVESGGGLVQPGGSLRLSCSASGSIWGIYHMGWYRQAP GKQRELVASITVDGSTHYADTVKGRFTISRDNSKNMVYLQM NSLRAEDTAVYYCDIRNVGGDYWGQGTQVTVSS | GSIWGIYH | ITVDGST | DIRNVGGDY |
| | SEQ ID NO: 27 | SEQ ID NO: 37 | SEQ ID NO: 46 | SEQ ID NO: 57 |
| Hu-HSA-PH-8-5 | QVQLVESGGGLVQPGGSLRLSCSASGSIWGIYHMGWYRQAP GKQRELVASITVDGSTHYADTVKGRFTISRDNSKNTLYLQM NSLEPEDTAVYYCDIRNVGGDYWGQGTQVTVSS | GSIWGIYH | ITVDGST | DIRNVGGDY |
| | SEQ ID NO: 78 | SEQ ID NO: 37 | SEQ ID NO: 46 | SEQ ID NO: 57 |

In this study, IMGT (http://www.imgt.org) was used to evaluate the humanization levels of HSA-59, HSA-M-27, HSA-PH-8 and humanized sequences. The results were shown in Table 8. The humanization levels of all humanized samples had been improved, which met the requirements of late-stage drug development.

**Table 8. Homology of humanized anti-HSA sequences with human sequences**

| No. | Germline | Homology |
|---|---|---|
| HSA-59 | GHV3-23 *04 | 79.4% |
| HSA-M-27 | IGHV3-23 *01 | 79.4% |
| HSA-PH-8 | IGHV3-64*04 | 69.8% |
| Hu-HSA-59-1 | IGHV3-23 *04 | 83.5% |
| Hu-HSA-59-2 | IGHV3-23 *04 | 85.6% |
| Hu-HSA-59-3 | IGHV3-23 *04 | 85.6% |
| Hu-HSA-M-27-1 | IGHV3-23 *01 | 83.5% |
| Hu-HSA-M-27-2 | IGHV3-23 *01 | 85.6% |
| Hu-HSA-M-27-3 | IGHV3-23 *01 | 85.6% |
| Hu-HSA-PH-8-1 | IGHV3-64*04 | 76% |
| Hu-HSA-PH-8-2 | IGHV3-64*04 | 78.1% |
| Hu-HSA-PH-8-3 | IGHV3-64*04 | 78.1% |
| Hu-HSA-PH-8-5 | IGHV3-64*04 | 78.1% |
| Hu-HSA-M-27-1-2 | IGHV3-23 *01 | 82.5% |

The protein construction, expression and purification methods were the same as in Example 2. The protein purity was obtained by HPLC detection, and the purity detection results were shown in Table 9. The HPLC was performed as follows, mobile phase: 150mM Na₂HPO₄•12H₂O, pH7.0; chromatographic conditions: detection wavelength: 280 nm; column temperature: 25°C; flow rate: 0.35 ml/min; detection time: 20 min; Zenix-C SEC-300 column (SEPAX 4.6×300mm, 3µm).

**Table 9. Purity detection results of humanized anti-HSA antibodies**

| No. | Monomer ratio (%) |
|---|---|
| Hu-HSA-59-1 | 98.30 |
| Hu-HSA-59-2 | 98.60 |
| Hu-HSA-59-3 | 99.50 |
| Hu-HSA-M-27-1 | 97.60 |
| Hu-HSA-M-27-2 | 97.90 |
| Hu-HSA-M-27-3 | 98.60 |
| Hu-HSA-PH-8-1 | 97.30 |
| Hu-HSA-PH-8-2 | 80.10 |
| Hu-HSA-PH-8-3 | 97.40 |
| Hu-HSA-PH-8-5 | 96.40 |
| Hu-HSA-M-27-1-2 | 98.10 |

### Example 5. Determination of affinity of humanized anti-HSA antibodies

### 5.1. Affinity determination of humanized antibodies

In this example, the binding affinity of the purified humanized anti-HSA antibodies with HSA was detected. The experimental method was the same as that of Example 3. The results were shown in Table 10. The results showed that the humanized anti-HSA antibodies had good binding activity with HSA protein.

**Table 10. Affinity of humanized molecules to HSA**

| No. | KD(M) | Kon(1/Ms) | Koff(1/s) |
|---|---|---|---|
| Hu-HSA-59-1 | 1.59E-07 | 8.22E+04 | 1.30E-02 |
| Hu-HSA-59-2 | 1.74E-06 | 1.24E+04 | 2.15E-02 |
| Hu-HSA-59-3 | 4.54E-07 | 3.29E+04 | 1.49E-02 |
| Hu-HSA-M-27-1 | 5.20E-08 | 1.38E+05 | 7.15E-03 |
| Hu-HSA-M-27-2 | 4.23E-08 | 1.34E+05 | 5.66E-03 |
| Hu-HSA-M-27-3 | 4.09E-08 | 1.69E+05 | 6.92E-03 |
| Hu-HSA-PH-8-1 | 7.28E-08 | 1.02E+05 | 7.44E-03 |
| Hu-HSA-PH-8-2 | 2.13E-07 | 5.32E+04 | 1.13E-02 |
| Hu-HSA-PH-8-3 | 6.06E-08 | 1.26E+05 | 7.64E-03 |
| Hu-HSA-PH-8-5 | 5.28E-08 | 1. 19E+05 | 6.27E-03 |
| Hu-HSA-M-27-1-2 | 3.48E-08 | 1.92E+05 | 6.66E-03 |

In this example, the binding affinity of the purified humanized anti-HSA antibodies with MSA/CSA was also detected. The experimental method was the same as that of Example 3. The experimental results were shown in Table 11.

**Table 11. Affinity of humanized molecules to MSA/CSA**

| No. | MSA | | | CSA | | |
|---|---|---|---|---|---|---|
| | KD(M) | Kon(1/Ms) | Koff(1/s) | KD(M) | Kon(1/Ms) | Koff(1/s) |
| Hu-HSA-59-1 | 1.04E-08 | 3.73E+05 | 3.86E-03 | 6.62E-08 | 2.80E+05 | 1.85E-02 |
| Hu-HSA-59-2 | 1.30E-08 | 3.54E+05 | 4.59E-03 | 8.04E-08 | 2.89E+05 | 2.33E-02 |
| Hu-HSA-59-3 | 1.17E-08 | 3.57E+05 | 4.19E-03 | 7.14E-08 | 2.73E+05 | 1.95E-02 |
| Hu-HSA-M-27-1 | 7.79E-09 | 5.05E+05 | 3.93E-03 | 4.04E-08 | 3.78E+05 | 1.53E-02 |
| Hu-HSA-M-27-2 | 8.21E-09 | 4.36E+05 | 3.58E-03 | 4.36E-08 | 2.99E+05 | 1.30E-02 |
| Hu-HSA-M-27-3 | 7.62E-09 | 5.17E+05 | 3.94E-03 | 3.77E-08 | 4.07E+05 | 1.53E-02 |
| Hu-HSA-PH-8-1 | 2.55E-08 | 2.68E+05 | 6.85E-03 | 1.62E-08 | 2.76E+05 | 4.46E-03 |
| Hu-HSA-PH-8-2 | 4.20E-08 | 1.99E+05 | 8.35E-03 | 3.28E-08 | 1.69E+05 | 5.54E-03 |
| Hu-HSA-PH-8-3 | 2.56E-08 | 2.56E+05 | 6.53E-03 | 1.78E-08 | 2.61E+05 | 4.64E-03 |
| Hu-HSA-PH-8-5 | 2.95E-08 | 2.41E+05 | 7.11E-03 | 1.25E-08 | 2.98E+05 | 3.73E-03 |
| Hu-HSA-M-27-1-2 | 6.22E-09 | 6.12E+05 | 3.81E-03 | 4.78E-08 | 3.14E+05 | 1.50E-02 |

### 5.2. Affinity determination of humanized antibodies at different pH values

The recycling mechanism of HSA is similar to that of IgG. HSA is internalized into vascular endothelial cells through pinocytosis. Within endosome, the binding of membrane-bound FcRn to HSA is facilitated at an endosomal pH of about 6.0; then the complex in endosome is recycled into the blood; and at pH 7.4, the membrane-bound FcRn and HSA quickly dissociate, allowing HSA to recirculate.

In this example, the affinity of the purified humanized anti-HSA antibodies to HSA under different pH conditions was detected. The experimental method was the same as that of Example 3. The experimental results were shown in Table 12. The results showed that the affinity of the humanized anti-HSA antibodies to HSA protein was good under different pH conditions.

**Table 12. Affinity of humanized molecules to HSA under different pH conditions**

| No. | Hu-HSA-M-27-1 | | |
|---|---|---|---|
| | KD(M) | Kon(1/Ms) | Koff(1/s) |
| pH5.5 | 2.99E-08 | 1.83E+05 | 5.47E-03 |
| pH6.5 | 1.43E-08 | 1.96E+05 | 2.81E-03 |
| pH7.4 | 5.04E-08 | 1.66E+05 | 8.36E-03 |

| No. | Hu-HSA-59-1 | | |
|---|---|---|---|
| pH5.5 | 4.44E-08 | 1.08E+05 | 4.78E-03 |
| pH6.5 | 9.15E-08 | 8.60E+04 | 7.86E-03 |
| pH7.4 | 1.63E-07 | 6.93E+04 | 1.13E-02 |

| No. | Hu-HSA-PH-8-5 | | |
|---|---|---|---|
| pH5.5 | 9.53E-09 | 1.74E+05 | 1.66E-03 |
| pH6.5 | 1.437E-08 | 1.39E+05 | 2.00E-03 |
| pH7.4 | 2.30E-08 | 1.32E+05 | 3.03E-03 |

### Example 6. Detection of half-life prolongation effect of anti-HSA nanobodies

### 6.1. Molecular construction and protein preparation

In order to confirm the half-life prolongation effect of anti-HSA nanobodies, in this example, multispecific antibodies were constructed. The multispecific antibodies Bi-340-352, Bi-340-353 and Bi-340-354 were each independently composed of peptide chain #1 and peptide chain #2, and contained three antigen-binding domains, i.e., a Fab targeting the first antigen, a nanobody (VHH1) targeting the second antigen, and a nanobody (VHH2) targeting the third antigen, wherein the VHH2 was Hu-HSA-M-27-1, Hu-HSA-59-1. Hu-HSA-PH-8-5 or non-HSA related VHH as a control. The structures and sequences of the multispecific antibodies were shown in Tables 13-1 and 13-2, wherein the domains in the peptide chain #1 and peptide chain # 2 were linked by a linker containing GGGGS.

**Table 13-1. Structures of multispecific antibodies**

| Name | Peptide chain #1 (Bi-352/353/354/356) | | | | Peptide chain #2 (Bi-340) | | |
|---|---|---|---|---|---|---|---|
| | Fab light | VHH1 | VHH2 | Full | Fab heavy | VHH1 | Full |
| | chain | | | length | chain | | length |
| Bi-340-352 | SEQ ID NO:79 | SEQ ID NO:81 | Hu-HSA-M-27-1 | SEQ ID NO: 69 | SEQ ID NO: 80 | SEQ ID NO:81 | SEQ ID NO: 76 |
| Bi-340-353 | | | Hu-HSA-59-1 | SEQ ID NO: 70 | | | |
| Bi-340-354 | | | Hu-HSA-PH-8-5 | SEQ ID NO:71 | | | |
| Bi-340-356 | | | Non-HSA related VHH (control) | SEQ ID NO:77 | | | |

**Table 13-2. Related sequences of multispecific antibodies**

| No. | Amino acid sequence |
|---|---|
| Bi-352 | DIVMTQSPDSLAVSLGERATINCKSSQSLLNARTGKNYLAWYQQKPGQ PPKLLIYWASTRESGVPDRFSGSGSGTDFTLTISSLQAEDVAVYYCKQS YSRRTFGGGTKVEIKRTVAAPSVFIFPPSDEQLKSGTASVVCLLNNFYPR EAKVQWKVDNALQSGNSQESVTEQDSKDSTYSLSSTLTLSKADYEKH KVYACEVTHQGLSSPVTKSFNRGECGGGGSGGGGSGGGGSGGGGSGQ VQLVESGGGLVQPGGSLRLSCSASGNSANIFSFASVAWYRQAPGKGLE LVSVITSAGGTKYSDSVKGRFTISRDNSKNTLYLQMNSLRAEDTAVYY CNVDYLQDYWGQGTLVTVSSGGGGSGGGGSGGGGSGGGGSGEVQLL ESGGGLVQPGGSLRLSCAASGFTLDYYAIGWFRQAPGKEREGVSCIASS GGSTNYADSVKGRFTISRDNSKNTVYLQMNSLKPEDTAVYYCAAAVL ECRTVVRGYDYWGQGTQVTVSS |
| | SEQ ID NO: 69 |
| Bi-353 | DIVMTQSPDSLAVSLGERATINCKSSQSLLNARTGKNYLAWYQQKPGQ PPKLLIYWASTRESGVPDRFSGSGSGTDFTLTISSLQAEDVAVYYCKQS YSRRTFGGGTKVEIKRTVAAPSVFIFPPSDEQLKSGTASVVCLLNNFYPR EAKVQWKVDNALQSGNSQESVTEQDSKDSTYSLSSTLTLSKADYEKH KVYACEVTHQGLSSPVTKSFNRGECGGGGSGGGGSGGGGSGGGGSGQ VQLVESGGGLVQPGGSLRLSCSASGNSANIFSFASVAWYRQAPGKGLE LVSVITSAGGTKYSDSVKGRFTISRDNSKNTLYLQMNSLRAEDTAVYY CNVDYLQDYWGQGTLVTVSSGGGGSGGGGSGGGGSGGGGSGEVQLL ESGGGLVQPGGSLRLSCAASGFTLDYYAIGWFRQAPGKEREEVSCISSS GGSTNYADSVKGRFTISRDNSKNTVYLQMNSLKPEDTAVYYCAAALLE CRVRSWPSDNWGQGTQVTVSS |
| | SEQ ID NO: 70 |
| Bi-354 | DIVMTQSPDSLAVSLGERATINCKSSQSLLNARTGKNYLAWYQQKPGQ PPKLLIYWASTRESGVPDRFSGSGSGTDFTLTISSLQAEDVAVYYCKQS YSRRTFGGGTKVEIKRTVAAPSVFIFPPSDEQLKSGTASVVCLLNNFYPR EAKVQWKVDNALQSGNSQESVTEQDSKDSTYSLSSTLTLSKADYEKH KVYACEVTHQGLSSPVTKSFNRGECGGGGSGGGGSGGGGSGGGGSGQ VQLVESGGGLVQPGGSLRLSCSASGNSANIFSFASVAWYRQAPGKGLE |
| | LVSVITSAGGTKYSDSVKGRFTISRDNSKNTLYLQMNSLRAEDTAVYY CNVDYLQDYWGQGTLVTVSSGGGGSGGGGSGGGGSGGGGSGQVQLV ESGGGLVQPGGSLRLSCSASGSIWGIYHMGWYRQAPGKQRELVASITV DGSTHYADTVKGRFTISRDNSKNTLYLQMNSLEPEDTAVYYCDIRNVG GDYWGQGTQVTVSS |
| | SEQ ID NO: 71 |
| Bi-340 | QVQLVQSGAEVKKPGASVKVSCKASGFNIKDYYMHWVRQAPGQRLE WMGWIDLEEGNTIYDAKFQGRVTITRDTSASTAYMELSSLRSEDTAVY YCARDAYGRYFYDVWGQGTLVTVSSASTKGPSVFPLAPSSKSTSGGTA ALGCLVKDYFPEPVTVSWNSGALTSGVHTFPAVLQSSGLYSLSSVVTVP SSSLGTQTYICNVNHKPSNTKVDKKVEPKSCDKTHTGGGGSGGGGSGG GGSGGGGSGQVQLVESGGGLVQPGGSLRLSCSASGNSANIFSFASVAW YRQAPGKGLELVSVITSAGGTKYSDSVKGRFTISRDNSKNTLYLQMNSL RAEDTAVYYCNVDYLQDYWGQGTLVTVSS |
| | SEQ ID NO: 76 |
| Bi-356 | DIVMTQSPDSLAVSLGERATINCKSSQSLLNARTGKNYLAWYQQKPGQ PPKLLIYWASTRESGVPDRFSGSGSGTDFTLTISSLQAEDVAVYYCKQS YSRRTFGGGTKVEIKRTVAAPSVFIFPPSDEQLKSGTASVVCLLNNFYPR EAKVQWKVDNALQSGNSQESVTEQDSKDSTYSLSSTLTLSKADYEKH KVYACEVTHQGLSSPVTKSFNRGECGGGGSGGGGSGGGGSGGGGSGQ VQLVESGGGLVQPGGSLRLSCSASGNSANIFSFASVAWYRQAPGKGLE LVSVITSAGGTKYSDSVKGRFTISRDNSKNTLYLQMNSLRAEDTAVYY CNVDYLQDYWGQGTLVTVSSGGGGSGGGGSGGGGSGGGGSGEVQLQ ESGGGLVQPGGSLRLSCAASGFTFSSYWMYWLRQAPGKGLEWVSSINS DSSSTYYRDSVKGRFTISRDNAKNTLYLQMNSLKSEDTAVYYCAKDPG GYAKGQGTQVTVSS |
| | SEQ ID NO: 77 |
| Anti-second antigen Fab light chain | DIVMTQSPDSLAVSLGERATINCKSSQSLLNARTGKNYLAWYQQKPGQ PPKLLIYWASTRESGVPDRFSGSGSGTDFTLTISSLQAEDVAVYYCKQS YSRRTFGGGTKVEIKRTVAAPSVFIFPPSDEQLKSGTASVVCLLNNFYPR EAKVQWKVDNALQSGNSQESVTEQDSKDSTYSLSSTLTLSKADYEKH KVYACEVTHQGLSSPVTKSFNRGEC |
| | SEQ ID NO: 79 |
| Anti-second antigen Fab heavy chain | QVQLVQSGAEVKKPGASVKVSCKASGFNIKDYYMHWVRQAPGQRLE WMGWIDLEEGNTIYDAKFQGRVTITRDTSASTAYMELSSLRSEDTAVY YCARDAYGRYFYDVWGQGTLVTVSSASTKGPSVFPLAPSSKSTSGGTA ALGCLVKDYFPEPVTVSWNSGALTSGVHTFPAVLQSSGLYSLSSVVTVP SSSLGTQTYICNVNHKPSNTKVDKKVEPKSCDKTHT |
| | SEQ ID NO: 80 |
| Anti-third antigen nanobody VHH | QVQLVESGGGLVQPGGSLRLSCSASGNSANIFSFASVAWYRQAPGKGL ELVSVITSAGGTKYSDSVKGRFTISRDNSKNTLYLQMNSLRAEDTAVY YCNVDYLQDYWGQGTLVTVSS |
| | SEQ ID NO: 81 |
| Hu-HSA-M -27-1 VHH | EVQLLESGGGLVQPGGSLRLSCAASGFTLDYYAIGWFRQAPGKEREGV SCIASSGGSTNYADSVKGRFTISRDNSKNTVYLQMNSLKPEDTAVYYC AAAVLECRTVVRGYDYWGQGTQVTVSS |
| | SEQ ID NO: 10 |
| Hu-HSA-59 -1 | EVQLLESGGGLVQPGGSLRLSCAASGFTLDYYAIGWFRQAPGKEREEV SCISSSGGSTNYADSVKGRFTISRDNSKNTVYLQMNSLKPEDTAVYYCA AALLECRVRSWPSDNWGQGTQVTVSS |
| | SEQ ID NO: 4 |
| Hu-HSA-P H-8-5 | QVQLVESGGGLVQPGGSLRLSCSASGSIWGIYHMGWYRQAPGKQREL VASITVDGSTHYADTVKGRFTISRDNSKNTLYLQMNSLEPEDTAVYYC DIRNVGGDYWGQGTQVTVSS |
| | SEQ ID NO: 78 |
| Non-HSA related nanobody VHH | EVQLQESGGGLVQPGGSLRLSCAASGFTFSSYWMYWLRQAPGKGLEW VSSINSDSSSTYYRDSVKGRFTISRDNAKNTLYLQMNSLKSEDTAVYYC AKDPGGYAKGQGTQVTVSS |
| | SEQ ID NO: 82 |

The gene sequences encoding the peptide chain #1 and peptide chain #2 of Bi-340-352, Bi-340-353, Bi-340-354 and Bi-340-356 were synthesized by GENEWIZ Company. Recombinase that catalyzes homologous recombination (purchased from Vazyme) was used to construct the encoding gene sequences into pCDNA3.1 vector linearized by *EcoR* I/*Not* I double enzyme digestion, respectively, in which the construction process was carried out in accordance with the product instructions. The homologous recombination product for peptide chain #1 and the homologous recombination product for peptide chain #2 of each of the multispecific antibodies were co-transformed into Top10 competent cells, coated on a plate with ampicillin, and cultured at 37°C overnight, and single clones were picked for sequencing.

The plasmid was transfected into Expi-CHO cells using the ExpiCHO ^{™} expression system kit (purchased from Thermo), in which the transfection process was carried out according to the product instructions. After 5 days of cell culture, the supernatant was collected and purified using a KappaSelect (GE) affinity chromatography column. The specific method was carried out as follows: the sample was filtered through a 0.2um sterile syringe filter PES with a syringe. The column was balanced with 5 column volumes of equilibrium buffer (20 mM PB + 0.15 M NaCl, pH 7.4) until the effluent conductivity and pH remained unchanged. Sample was loaded at a flow rate of 0.5 ml/min. After loading, the column was continuously rinsed with equilibrium buffer until penetration was complete and the UV value no longer decreased. Elution buffer (0.1 M glycine-HCl, pH 3.0) was used for elution, and the effluent was collected. After elution, the collected antibody solution should be immediately neutralized with an alkaline buffer (e.g., 1 M Tris/HCl, pH 8.0) to the pH at which the antibody was stable.

The purity of the obtained protein was detected by HPLC. The HPLC was carried out as follows, mobile phase: 150mM Na₂HPO₄•12H₂O, pH7.0; chromatographic conditions: detection wavelength: 280 nm, column temperature: 25°C, flow rate: 0.35 ml/min, detection time: 20 min, Zenix-C SEC-300 column (SEPAX 4.6×300mm, 3µm).

### 6.2. Detection of protein half-life

The injection dose for mice was 10 mg/kg. Blood sampling time points: 3 minutes, 30 minutes, 2 hours, 6 hours, 24 hours, 48 hours, 96 hours, and 168 hours after administration. Whole blood sample was allowed to stand at 2-8°C for 30 minutes, the serum was collected by centrifugation at 12000 rpm for 5 minutes, and the obtained serum was centrifuged at 2-8°C and 12000 rpm for 5 minutes, and stored at -80°C. The concentration of free antibodies in the serum was detected by ELISA. The results were shown in Fig. 1. The results showed that the half-life of the molecules with anti-HSA nanobodies in mice was about 23.7 hours (Bi-340-352), 24.2 hours (Bi-340-353) and 10.9 hours (Bi- 340-354), respectively; while the half-life of the molecule with non-HSA related nanobody (Bi-340-356) was about 1.4 hours. The results showed that the anti-HSA nanobodies could significantly increase the in vivo half-life of the molecules.

Although the specific embodiments of the present application have been described in detail, those skilled in the art will understand that various modifications and changes can be made to the details based on all the teachings that have been disclosed, and these changes are all within the scope of protection of the present application. The entirety of the present application is given by the appended claims and any equivalents thereof.

## Claims

1. A nanobody or antigen-binding fragment thereof capable of specifically binding to serum albumin, wherein the nanobody or antigen-binding fragment thereof comprises:
(a) a CDR1 having a structure selected from the group consisting of: X₁X₂X₃LX₄YYX₅ (Formula I, SEQ ID NO: 72), SGFTLDYYA (SEQ ID NO: 30), GSIWGIYH (SEQ ID NO: 37), GFTFSIYS (SEQ ID NO: 31), GSIFTFYR (SEQ ID NO: 33);
(b) a CDR2 having a structure selected from the group consisting of: IX₆SSGGX₇X₈ (Formula II, SEQ ID NO: 73), ITVDGST (SEQ ID NO: 46), ISSGGSP (SEQ ID NO: 40), ITTDTST (SEQ ID NO: 42);
(c) a CDR3 having a structure selected from the group consisting of: AAAX₉LECRTX₁₀X₁₁X₁₂X₁₃YX₁₄Y (Formula III, SEQ ID NO: 74), AAATX₁₅ECRGRSSSYDY (Formula IV, SEQ ID NO: 75), AAALLECRVRSWPSDN (SEQ ID NO: 49), AAAVLECRAAEYVNS (SEQ ID NO: 54), DIRNVGGDY (SEQ ID NO: 57), SPITSIFKA (SEQ ID NO: 48), NVRNVERDY (SEQ ID NO: 50);
wherein,
X₁ is selected from the group consisting of (i) amino acid residues G, R and (ii) amino acid residues that are conservative substitutions relative to (i);
X₂ is selected from the group consisting of (i) amino acid residues F, A, S, E and (ii) amino acid residues that are conservative substitutions relative to (i);
X₃ is selected from the group consisting of (i) amino acid residues T, Q and (ii) amino acid residues that are conservative substitutions relative to (i);
X₄ is selected from the group consisting of (i) amino acid residues D, E and (ii) amino acid residues that are conservative substitutions relative to (i);
X₅ is selected from the group consisting of (i) amino acid residue A and (ii) amino acid residues that are conservative substitutions relative to (i);
X₆ is selected from the group consisting of (i) amino acid residues A, S and (ii) amino acid residues that are conservative substitutions relative to (i);
X₇ is selected from the group consisting of (i) amino acid residues A, S and (ii) amino acid residues that are conservative substitutions relative to (i);
X₈ is selected from the group consisting of (i) amino acid residue T and (ii) amino acid residues that are conservative substitutions relative to (i);
X₉ is selected from the group consisting of (i) amino acid residues T, V and (ii) amino acid residues that are conservative substitutions relative to (i);
X₁₀ is selected from the group consisting of (i) amino acid residues T, V and (ii) amino acid residues that are conservative substitutions relative to (i);
X₁₁ is selected from the group consisting of (i) amino acid residues V, I, L and (ii) amino acid residues that are conservative substitutions relative to (i);
X₁₂ is selected from the group consisting of (i) amino acid residues R, V, T and (ii) amino acid residues that are conservative substitutions relative to (i);
X₁₃ is selected from the group consisting of (i) amino acid residues G, E and (ii) amino acid residues that are conservative substitutions relative to (i);
X₁₄ is selected from the group consisting of (i) amino acid residues D, A and (ii) amino acid residues that are conservative substitutions relative to (i);
X₁₅ is selected from the group consisting of (i) amino acid residues L, F and (ii) amino acid residues that are conservative substitutions relative to (i);
preferably, the nanobody or antigen-binding fragment thereof comprises a CDR1 as set forth in any one of SEQ ID NOs: 30-38; a CDR2 as set forth in any one of SEQ ID NOs: 39-46; and a CDR3 as set forth in any one of SEQ ID NOs: 47-59.

2. The nanobody or antigen-binding fragment thereof according to claim 1, wherein the nanobody or antigen-binding fragment thereof comprises:
(a) a CDR1, which has a sequence as shown in X₁X₂X₃LX₄YYX₅ (Formula I, SEQ ID NO: 72) or SGFTLDYYA (SEQ ID NO: 30);
(b) a CDR2, which has a structure as shown in IX₆SSGGX₇X₈ (Formula II, SEQ ID NO: 73);
(c) a CDR3, which has a structure selected from the group consisting of: AAAX₉LECRTX₁₀X₁₁X₁₂X₁₃YX₁₄Y (Formula III, SEQ ID NO: 74), AAATX₁₅ECRGRSSSYDY (Formula IV, SEQ ID NO: 75), AAALLECRVRSWPSDN (SEQ ID NO: 49), AAAVLECRAAEYVNS (SEQ ID NO: 54);
wherein,
X₁ is selected from the group consisting of (i) amino acid residues G, R and (ii) amino acid residues that are conservative substitutions relative to (i);
X2 is selected from the group consisting of (i) amino acid residues F, A, S, E and (ii) amino acid residues that are conservative substitutions relative to (i);
X3 is selected from the group consisting of (i) amino acid residues T, Q and (ii) amino acid residues that are conservative substitutions relative to (i);
X4 is selected from the group consisting of (i) amino acid residues D, E and (ii) amino acid residues that are conservative substitutions relative to (i);
X5 is selected from the group consisting of (i) amino acid residue A and (ii) amino acid residues that are conservative substitutions relative to (i). Base;
X6 is selected from the group consisting of (i) amino acid residues A, S and (ii) amino acid residues that are conservative substitutions relative to (i);
X7 is selected from the group consisting of (i) amino acid residues A, S and (ii) amino acid residues that are conservative substitutions relative to (i);
X8 is selected from the group consisting of (i) amino acid residue T and (ii) amino acid residues that are conservative substitutions relative to (i);
X9 is selected from the group consisting of (i) amino acid residues T, V and (ii) amino acid residues that are conservative substitutions relative to (i);
X10 is selected from the group consisting of (i) amino acid residues T, V and (ii) amino acid residues that are conservative substitutions relative to (i);
X11 is selected from the group consisting of (i) amino acid residues V, I, L and (ii) amino acid residues that are conservative substitutions relative to (i);
X12 is selected from the group consisting of (i) amino acid residues R, V, T and (ii) amino acid residues that are conservative substitutions relative to (i);
X13 is selected from the group consisting of (i) amino acid residues G, E and (ii) amino acid residues that are conservative substitutions relative to (i);
X14 is selected from the group consisting of (i) amino acid residues D, A and (ii) amino acid residues that are conservative substitutions relative to (i);
X15 is selected from the group consisting of (i) amino acid residues L, F and (ii) amino acid residues that are conservative substitutions relative to (i);
preferably, X1 is selected from the group consisting of amino acid residues G and R; X2 is selected from the group consisting of amino acid residues F, A, S and E; X3 is selected from the group consisting of amino acid residues T and Q; X4 is selected from the group consisting of amino acid residues D and E; X5 is amino acid residue A; X6 is selected from the group consisting of amino acid residues A and S; X7 is selected from amino acid residues A and S; X8 is amino acid residue T; X9 is selected from the group consisting of amino acid residues T and V; X10 is selected from the group consisting of amino acid residues T and V; X11 is selected from the group consisting of amino acid residues V, I and L; X12 is selected from the group consisting of amino acid residue R, V and T; X13 is selected from the group consisting of amino acid residue G and E; X14 is selected from the group consisting of amino acid residue D and A; X15 is selected from the group consisting of amino acid residues L and F;
preferably, the nanobody or antigen-binding fragment thereof comprises: a CDR1 as set forth in any one of SEQ ID NOs: 30, 32, 34-36, 38; a CDR2 as set forth in any one of SEQ ID NOs: 39, 41, 43-45; and a CDR3 as set forth in any of SEQ ID NOs: 47, 49, 51-56, 58-59.

3. The nanobody or antigen-binding fragment thereof according to claim 1 or 2, wherein the nanobody or antigen-binding fragment thereof comprises:
(a) a CDR1, which has a structure as shown in X₁X₂X₃LX₄YYX₅ (Formula I, SEQ ID NO: 72) or SGFTLDYYA (SEQ ID NO: 30);
(b) a CDR2, which has a structure as shown in IX₆SSGGX₇X₈ (Formula II, SEQ ID NO: 73);
(c) a CDR3, which has a structure selected from the group consisting of AAAX₉LECRTX₁₀X₁₁X₁₂X₁₃YX₁₄Y (Formula III, SEQ ID NO: 74), AAATX₁₅ECRGRSSSYDY (Formula IV, SEQ ID NO: 75), AAALLECRVRSWPSDN (SEQ ID NO: 49), AAAVLECRAAEYVNS (SEQ ID NO: 54);
wherein,
X₁ is selected from the group consisting of (i) amino acid residues G, R and (ii) amino acid residues that are conservative substitutions relative to (i);
X₂ is selected from the group consisting of (i) amino acid residues F, E, S and (ii) amino acid residues that are conservative substitutions relative to (i);
X₃ is selected from the group consisting of (i) amino acid residue T and (ii) amino acid residues that are conservative substitutions relative to (i);
X₄ is selected from the group consisting of (i) amino acid residue D and (ii) amino acid residues that are conservative substitutions relative to (i);
X5 is selected from the group consisting of (i) amino acid residue A and (ii) amino acid residues that are conservative substitutions relative to (i);
X6 is selected from the group consisting of (i) amino acid residues A, S and (ii) amino acid residues that are conservative substitutions relative to (i);
X7 is selected from the group consisting of (i) amino acid residues A, S and (ii) amino acid residues that are conservative substitutions relative to (i);
X8 is selected from the group consisting of (i) amino acid residue T and (ii) amino acid residues that are conservative substitutions relative to (i);
X9 is selected from the group consisting of (i) amino acid residues T, V and (ii) amino acid residues that are conservative substitutions relative to (i);
X10 is selected from the group consisting of (i) amino acid residue V and (ii) amino acid residues that are conservative substitutions relative to (i);
X11 is selected from the group consisting of (i) amino acid residues V, I and (ii) amino acid residues that are conservative substitutions relative to (i);
X12 is selected from the group consisting of (i) amino acid residues R, V and (ii) amino acid residues that are conservative substitutions relative to (i);
X13 is selected from the group consisting of (i) amino acid residues G, E and (ii) amino acid residues that are conservative substitutions relative to (i);
X14 is selected from the group consisting of (i) amino acid residue D and (ii) amino acid residues that are conservative substitutions relative to (i);
X15 is selected from the group consisting of (i) amino acid residues L, F and (ii) amino acid residues that are conservative substitutions relative to (i);
preferably, X1 is selected from the group consisting of amino acid residues G and R; X2 is selected from the group consisting of amino acid residues F, S and E; X3 is amino acid residue T; X4 is amino acid residue D; X5 is amino acid residue A; X6 is selected from the group consisting of amino acid residues A and S; X7 is selected from the group consisting of amino acid residues A and S; X8 is amino acid residue T; X9 is selected from the group consisting of amino acid residues T and V; X10 is amino acid residue V; X11 is selected from the group consisting of amino acid residues V and I; X12 is selected from the group consisting of amino acid residues R and V; X13 is selected from the group consisting of amino acid residues G and E; X14 is amino acid residue D; X15 is selected from the group consisting of amino acid residues L and F;
preferably, the nanobody or antigen-binding fragment thereof comprises: a CDR1 as set forth in any one of SEQ ID NOs: 30, 32, 34-36; a CDR2 as set forth in any one of SEQ ID NOs: 39, 41, 43-45; and a CDR3 as set forth in any one of SEQ ID NOs: 47, 49, 51, 52, 54-56, 58.

4. The nanobody or antigen-binding fragment thereof according to claim 1, wherein the nanobody or antigen-binding fragment thereof comprises:
(a) a CDR1, which has: a sequence as set forth in SEQ ID NO: 32, or a sequence having a substitution, deletion or addition of one or several amino acids (e.g., a substitution, deletion or addition of 1, 2 or 3 amino acids) as compared to the sequence as set forth in SEQ ID NO: 32;
(b) a CDR2, which has: a sequence as set forth in SEQ ID NO: 41, or a sequence having a substitution, deletion or addition of one or several amino acids (e.g., a substitution, deletion or addition of 1, 2 or 3 amino acids) as compared to the sequence as set forth in SEQ ID NO: 41; and
(c) a CDR3, which has: a sequence as set forth in SEQ ID NO: 49, or a sequence having a substitution, deletion or addition of one or several amino acids (e.g., a substitution, deletion or addition of 1, 2 or 3 amino acids) as compared to the sequence as set forth in SEQ ID NO: 49;
preferably, the substitution is a conservative substitution;
preferably, the nanobody or antigen-binding fragment thereof comprises: a CDR1 as set forth in SEQ ID NO: 32, a CDR2 as set forth in SEQ ID NO: 41, and a CDR3 as set forth in SEQ ID NO: 49.

5. The nanobody or antigen-binding fragment thereof according to claim 1, wherein the nanobody or antigen-binding fragment thereof comprises:
(a) a CDR1, which has: a sequence as set forth in SEQ ID NO: 32 or 34, or a sequence having a substitution, deletion or addition of one or several amino acids (e.g., a substitution, deletion or addition of 1, 2 or 3 amino acids) as compared to the sequence as set forth in SEQ ID NO: 32 or 34;
(b) a CDR2, which has: a sequence as set forth in SEQ ID NO: 43, or a sequence having a substitution, deletion or addition of one or several amino acids (e.g., a substitution, deletion or addition of 1, 2 or 3 amino acids) as compared to the sequence as set forth in SEQ ID NO: 43; and
(c) a CDR3, which has: a sequence as set forth in SEQ ID NO: 52, or a sequence having a substitution, deletion or addition of one or several amino acids (e.g., a substitution, deletion or addition of 1, 2 or 3 amino acids) as compared to the sequence as set forth in SEQ ID NO: 52;
preferably, the substitution is a conservative substitution;
preferably, the nanobody or antigen-binding fragment thereof comprises:
(a) a CDR1, which has a structure as shown in X1X2X3LX4YYX5 (Formula I, SEQ ID NO: 72), wherein X1 is amino acid residue G; X2 is selected from the group consisting of (i) amino acid residues F, E and (ii) amino acid residues that are conservative substitutions relative to (i); X3 is amino acid residue T; X4 is amino acid residue D; X5 is amino acid residue A;
(b) a CDR2, which has a structure as shown in IASSGGST (SEQ ID NO: 43);
(c) a CDR3, which has a structure as shown in AAAVLECRTVVRGYDY (SEQ ID NO: 52);
preferably, the nanobody or antigen-binding fragment thereof comprises: a CDR1 as set forth in SEQ ID NO: 32 or 34; a CDR2 as set forth in SEQ ID NO: 43; and a CDR3 as set forth in SEQ ID NO: 52.

6. The nanobody or antigen-binding fragment thereof according to claim 1, wherein the nanobody or antigen-binding fragment thereof comprises:
(a) a CDR1, which has: a sequence as set forth in SEQ ID NO: 37, or a sequence having a substitution, deletion or addition of one or several amino acids (e.g., a substitution, deletion or addition of 1, 2 or 3 amino acids) as compared to the sequence as set forth in SEQ ID NO: 37;
(b) a CDR2, which has: a sequence as set forth in SEQ ID NO: 46, or a sequence having a substitution, deletion or addition of one or several amino acids (e.g., a substitution, deletion or addition of 1, 2 or 3 amino acids) as compared to the sequence as set forth in SEQ ID NO: 46; and
(c) a CDR3, which has: a sequence as set forth in SEQ ID NO: 57, or a sequence having a substitution, deletion or addition of one or several amino acids (e.g., a substitution, deletion or addition of 1, 2 or 3 amino acids) as compared to the sequence as set forth in SEQ ID NO: 57;
preferably, the substitution is a conservative substitution;
preferably, the nanobody or antigen-binding fragment thereof comprises: a CDR1 as set forth in SEQ ID NO: 37; a CDR2 as set forth in SEQ ID NO: 46, and a CDR3 as set forth in SEQ ID NO: 57.

7. The nanobody or antigen-binding fragment thereof according to claim 1, wherein the nanobody or antigen-binding fragment thereof comprises:
(a) a CDR1, which has: a sequence as set forth in SEQ ID NO: 31, or a sequence having a substitution, deletion or addition of one or several amino acids (e.g., a substitution, deletion or addition of 1, 2 or 3 amino acids) as compared to the sequence as set forth in SEQ ID NO: 31;
(b) a CDR2, which has: a sequence as set forth in SEQ ID NO: 40, or a sequence having a substitution, deletion or addition of one or several amino acids (e.g., a substitution, deletion or addition of 1, 2 or 3 amino acids) as compared to the sequence as set forth in SEQ ID NO: 40; and
(c) a CDR3, which has: a sequence as set forth in SEQ ID NO: 48, or a sequence having a substitution, deletion or addition of one or several amino acids (e.g., a substitution, deletion or addition of 1, 2 or 3 amino acids) as compared to the sequence as set forth in SEQ ID NO: 48;
preferably, the substitution is a conservative substitution;
preferably, the nanobody or antigen-binding fragment thereof comprises: a CDR1 as set forth in SEQ ID NO: 31, a CDR2 as set forth in SEQ ID NO: 40, and a CDR3 as set forth in SEQ ID NO: 48.

8. The nanobody or antigen-binding fragment thereof according to claim 1, wherein the nanobody or antigen-binding fragment thereof comprises:
(a) a CDR1, which has: a sequence as set forth in SEQ ID NO: 33, or a sequence having a substitution, deletion or addition of one or several amino acids (e.g., a substitution, deletion or addition of 1, 2 or 3 amino acids) as compared to the sequence as set forth in SEQ ID NO: 33;
(b) a CDR2, which has: a sequence as set forth in SEQ ID NO: 42, or a sequence having a substitution, deletion or addition of one or several amino acids (e.g., a substitution, deletion or addition of 1, 2 or 3 amino acids) as compared to the sequence as set forth in SEQ ID NO: 42; and
(c) a CDR3, which has: a sequence as set forth in SEQ ID NO: 50, or a sequence having a substitution, deletion or addition of one or several amino acids (e.g., a substitution, deletion or addition of 1, 2 or 3 amino acids) as compared to the sequence as set forth in SEQ ID NO: 50;
preferably, the substitution is a conservative substitution;
preferably, the nanobody or antigen-binding fragment thereof comprises: a CDR1 as set forth in SEQ ID NO: 33, a CDR2 as set forth in SEQ ID NO: 42, and a CDR3 as set forth in SEQ ID NO: 50.

9. The nanobody or antigen-binding fragment thereof according to any one of claims 1 to 8, comprising:
(1) a CDR1 as set forth in SEQ ID NO: 32; a CDR2 as set forth in SEQ ID NO: 41; and a CDR3 as set forth in SEQ ID NO: 49;
(2) a CDR1 as set forth in SEQ ID NO: 32 or 34; a CDR2 as set forth in SEQ ID NO: 43; and a CDR3 as set forth in SEQ ID NO: 52;
(3) a CDR1 as set forth in SEQ ID NO: 37; a CDR2 as set forth in SEQ ID NO: 46; and, a CDR3 as set forth in SEQ ID NO: 57;
(4) a CDR1 as set forth in SEQ ID NO: 30; a CDR2 as set forth in SEQ ID NO: 39; and, a CDR3 as set forth in SEQ ID NO: 47;
(5) a CDR1 as set forth in SEQ ID NO: 31; a CDR2 as set forth in SEQ ID NO: 40; and, a CDR3 as set forth in SEQ ID NO: 48;
(6) a CDR1 as set forth in SEQ ID NO: 33; a CDR2 as set forth in SEQ ID NO: 42; and, a CDR3 as set forth in SEQ ID NO: 50;
(7) a CDR1 as set forth in SEQ ID NO: 32; a CDR2 as set forth in SEQ ID NO: 43; and, a CDR3 as set forth in SEQ ID NO: 51;
(8) a CDR1 as set forth in SEQ ID NO: 35; a CDR2 as set forth in SEQ ID NO: 43; and, a CDR3 as set forth in SEQ ID NO: 52;
(9) a CDR1 as set forth in SEQ ID NO: 32; a CDR2 as set forth in SEQ ID NO: 43; and, a CDR3 as set forth in SEQ ID NO: 53;
(10) a CDR1 as set forth in SEQ ID NO: 36; a CDR2 as set forth in SEQ ID NO: 41; and, a CDR3 as set forth in SEQ ID NO: 54;
(11) a CDR1 as set forth in SEQ ID NO: 35; a CDR2 as set forth in SEQ ID NO: 44; and, a CDR3 as set forth in SEQ ID NO: 55;
(12) a CDR1 as set forth in SEQ ID NO: 32; a CDR2 as set forth in SEQ ID NO: 45; and, a CDR3 as set forth in SEQ ID NO: 56;
(13) a CDR1 as set forth in SEQ ID NO: 32; a CDR2 as set forth in SEQ ID NO: 41; and, a CDR3 as set forth in SEQ ID NO: 52;
(14) a CDR1 as set forth in SEQ ID NO: 32; a CDR2 as set forth in SEQ ID NO: 41; and, a CDR3 as set forth in SEQ ID NO: 58; or
(15) a CDR1 as set forth in SEQ ID NO: 38; a CDR2 as set forth in SEQ ID NO: 41; and, a CDR3 as set forth in SEQ ID NO: 59.

10. The nanobody or antigen-binding fragment thereof according to any one of claims 1 to 9, which comprises an amino acid sequence selected from the group consisting of:
(i) a sequence as set forth in any one of SEQ ID NOs: 3, 9, 24, 1-2, 7-8, 14-23, 28-29;
(ii) a sequence having a substitution, deletion or addition of one or several amino acids (e.g., a substitution, deletion or addition of 1, 2, 3, 4 or 5 amino acids) as compared to the sequence as set forth in any one of SEQ ID NOs: 3, 9, 24, 1-2, 7-8, 14-23, 28-29; and
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 3, 9, 24, 1-2, 7-8, 14-23, 28-29;
preferably, the substitution is a conservative substitution.

11. The nanobody or antigen-binding fragment thereof according to any one of claims 1 to 9, wherein the nanobody or antigen-binding fragment thereof is humanized;
preferably, the nanobody or antigen-binding fragment thereof further comprises a heavy chain framework region of a human immunoglobulin (e.g., a heavy chain framework region contained in an amino acid sequence encoded by a human heavy chain germline antibody gene), wherein the heavy chain framework region optionally comprises one or more (e.g., 1, 2, 3, 4, 5, 6, 7, 8, 9 or 10) back mutations from human residues to camelid residues.

12. The nanobody or antigen-binding fragment thereof according to claim 11, wherein the nanobody or antigen-binding fragment thereof comprises an amino acid sequence selected from the group consisting of:
(i) a sequence as set forth in any one of SEQ ID NOs: 4-6, 10-13, 25-27, 78;
(ii) a sequence having a substitution, deletion or addition of one or several amino acids (e.g., a substitution, deletion or addition of 1, 2, 3, 4 or 5 amino acids) as compared to the sequence as set forth in any one of SEQ ID NOs: 4-6, 10-13, 25-27, 78; and
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in any one of SEQ ID NOs: 4-6, 10-13, 25-27, 78;
preferably, the substitution is a conservative substitution.

13. The nanobody or antigen-binding fragment thereof according to any one of claims 1 to 12, wherein the serum albumin is selected from the group consisting of human serum albumin (HSA), mouse serum albumin (MSA) and/or cynomolgus serum albumin (CSA);
preferably, the binding of the nanobody or antigen-binding fragment thereof to the serum albumin is pH-independent;
preferably, the nanobody or antigen-binding fragment thereof is capable of specifically binding to the serum albumin in the pH range of 5 to 8 (e.g., 5.5 to 7.4).

14. A polypeptide construct capable of specifically binding to serum albumin, which comprises the nanobody or antigen-binding fragment thereof according to any one of claims 1 to 13, and an immunoglobulin Fc domain;
preferably, the immunoglobulin Fc domain is connected to the N-terminal and/or C-terminal (e.g., C-terminal) of the nanobody or antigen-binding fragment thereof, optionally via a peptide linker;
preferably, the immunoglobulin Fc domain is an Fc domain of IgG (e.g., an Fc domain of IgG1);
preferably, the immunoglobulin Fc domain comprises a sequence as set forth in SEQ ID NO: 68, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or several amino acids (e.g., a substitution, deletion, or addition of 1, 2, 3, 4, or 5 amino acids) as compared thereto.

15. The polypeptide construct according to claim 14, wherein the serum albumin is selected from the group consisting of human serum albumin (HSA), mouse serum albumin (MSA) and/or cynomolgus serum albumin (CSA);
preferably, the binding of the polypeptide construct to the serum albumin is pH-independent;
preferably, the polypeptide construct is capable of specifically binding to the serum albumin in the pH range of 5 to 8 (e.g., 5.5 to 7.4).

16. A fusion protein, which comprises the nanobody or antigen-binding fragment thereof according to any one of claims 1 to 13 or the polypeptide construct according to claim 13 or 14, and an additional peptide domain;
preferably, the additional peptide domain is selected from a polypeptide domains having a therapeutic effect;
preferably, the additional polypeptide domain is connected to the N-terminal and/or C-terminal of the nanobody or antigen-binding fragment thereof or the polypeptide construct, optionally via a peptide linker.

17. An isolated nucleic acid molecule, which encodes the nanobody or antigen-binding fragment thereof according to any one of claims 1 to 13, the polypeptide construct according to claim 14 or 15, or the fusion protein according to claim 16.

18. A vector, which comprises the nucleic acid molecule according to claim 17; preferably, the vector is a cloning vector or an expression vector.

19. A host cell, which comprises the nucleic acid molecule according to claim 17 or the vector according to claim 18.

20. A method for preparing the nanobody or antigen-binding fragment thereof according to any one of claims 1 to 13, the polypeptide construct according to claim 14 or 15, or the fusion protein according to claim 16, comprising culturing the host cell according to claim 19 under a condition that allows protein expression, and recovering the nanobody or antigen-binding fragment thereof or the polypeptide construct or the fusion protein from a culture of the cultured host cell.

21. A bispecific or multispecific antibody, which comprises the nanobody or antigen-binding fragment thereof according to any one of claims 1 to 13 or the polypeptide construct according to claim 14 or 15;
preferably, the bispecific or multispecific antibody is capable of specifically binding to serum albumin and additionally specifically binding to one or more other targets;
preferably, the bispecific or multispecific antibody further comprises at least one second antibody having a binding specificity for a second target;
preferably, the bispecific or multispecific antibody comprises:
(i) the nanobody or antigen-binding fragment thereof according to any one of claims 1 to 13 or the polypeptide construct according to claim 14 or 15 (e.g., a nanobody or antigen-binding fragment thereof or polypeptide construct comprising a sequence as set forth in any one of SEQ ID NOs: 10, 4, 78) as a first antigen-specific binding domain;
(ii) a second antigen-specific binding domain (e.g., a Fab) comprising a light chain as set forth in SEQ ID NO: 79 and a heavy chain as set forth in SEQ ID NO: 80;
and,
(iii) a third antigen-specific binding domain (e.g., a nanobody VHH) as set forth in SEQ ID NO: 81;
preferably, the bispecific or multispecific antibody comprises a first polypeptide chain and a second polypeptide chain, wherein the first polypeptide chain comprises (e.g., from N-terminal to C-terminal): a light chain or heavy chain (e.g., VH and CH1) of the second antigen-specific binding domain, the third antigen-specific binding domain, the first antigen-specific binding domain; and the second polypeptide chain comprises (e.g., from N-terminal to C-terminal): a heavy chain (e.g., VH and CH1) or light chain of the second antigen-specific binding domain, the third antigen-specific binding domain; wherein the first polypeptide chain is paired with the second polypeptide chain to form a complete second antigen-specific binding domain; preferably, each domain in the first polypeptide chain and/or the second polypeptide chain is connected by a linker (e.g., a peptide linker comprising one or more glycines and/or one or more serines);
preferably, the bispecific or multispecific antibody comprises:
(1) a first peptide having a sequence as set forth in SEQ ID NO: 69, and, a second peptide having a sequence as set forth in SEQ ID NO: 76;
(2) a first peptide having a sequence as set forth in SEQ ID NO: 70, and, a second peptide having a sequence as set forth in SEQ ID NO: 76;
or,
(3) a first peptide having a sequence as set forth in SEQ ID NO: 71, and, a second peptide having a sequence as set forth in SEQ ID NO: 76;
preferably, the first peptide is present in the first peptide chain, and the second peptide is present in the second peptide chain; preferably, the first peptide chain and the second peptide chain form a complex.

22. A conjugate, which comprises the nanobody or antigen-binding fragment thereof according to any one of claims 1 to 13 or the polypeptide construct according to claim 14 or 15, and a therapeutic agent linked to the nanobody or antigen-binding fragment thereof or polypeptide construct;
preferably, the therapeutic agent is selected from the group consisting of anti-tumor drugs, such as cytotoxic agents, hormone drugs, biological response modifiers, and other antibodies or their antigen-binding fragments.

23. A pharmaceutical composition, which comprises the nanobody or antigen-binding fragment thereof according to any one of claims 1 to 13, the polypeptide construct according to claim 14 or 15, the fusion protein according to claim 16, the isolated nucleic acid molecule according to claim 17, the vector according to claim 18, the host cell according to claim 19, the bispecific or multispecific antibody according to claim 21, or the conjugate according to claim 22;
preferably, the pharmaceutical composition further comprises a pharmaceutically acceptable carrier and/or excipient;
preferably, the pharmaceutical composition comprises the nanobody or antigen-binding fragment thereof according to any one of claims 1 to 13, the polypeptide construct according to claim 14 or 15, or a nucleic acid molecule, vector or host cell encoding the nanobody or antigen-binding fragment or polypeptide construct;
preferably, the pharmaceutical composition comprises the fusion protein according to claim 16, or a nucleic acid molecule, vector or host cell encoding the fusion protein;
preferably, the pharmaceutical composition comprises the bispecific or multispecific antibody according to claim 21, or a nucleic acid molecule, vector or host cell encoding the bispecific or multispecific antibody;
preferably, the pharmaceutical composition comprises the conjugate according to claim 22.

24. Use of the nanobody or antigen-binding fragment thereof according to any one of claims 1 to 13, the polypeptide construct according to claim 14 or 15, the fusion protein according to claim 16, the isolated nucleic acid molecule according to claim 17, the vector according to claim 18, the host cell according to claim 19, the bispecific or multispecific antibody according to claim 21, or the conjugate according to claim 22, or the pharmaceutical composition according to claim 23 in the manufacture of a medicament;
preferably, the medicament is capable of directly or indirectly participating in FcRn-mediated serum albumin circulation in a subject;
preferably, the medicament shows an prolongated in vivo half-life relative to the corresponding drug lacking the nanobody or antigen-binding fragment thereof;
preferably, the medicament is a protein drug;
preferably, the subject is a mammal, such as a human, a mouse or a cynomolgus monkey.

25. A method for prolongation of in vivo half-life of a drug, comprising: linking the nanobody or antigen-binding fragment thereof according to any one of claims 1 to 13 or the polypeptide construct according to claim 14 or 15 to the drug;
preferably, the prolongation of in vivo half-life is relative to the in vivo half-life of the drug in the absence of the nanobody or antigen-binding fragment thereof;
preferably, the drug is selected from the group consisting of macromolecular drugs (e.g., polypeptide drugs);
preferably, the subject is a mammal, such as a human, a mouse or a cynomolgus monkey.
